(19) Europäisches Patentamt
European Patent Office
Office européen des brevets

(11) **EP 4 186 918 A1**

(12) **EUROPEAN PATENT APPLICATION**

(43) Date of publication:
**31.05.2023 Bulletin 2023/22**

(21) Application number: **21211241.1**

(22) Date of filing: **30.11.2021**

(51) International Patent Classification (IPC):
**C07K 14/47** (2006.01)    **C07K 7/06** (2006.01)
**A61K 38/08** (2019.01)    **A61K 38/17** (2006.01)
**A61P 25/28** (2006.01)    **G01N 33/68** (2006.01)

(52) Cooperative Patent Classification (CPC):
**A61K 38/08; A61K 38/1716; A61P 25/28;**
**C07K 7/06; C07K 14/4711; G01N 33/68**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicants:
- **Université de Rennes**
  **35042 Rennes (FR)**
- **Ecole des Hautes Etudes en Santé Publique**
  **(EHESP)**
  **35043 Rennes cedex (FR)**
- **Institut National de la Santé et de la**
  **Recherche Médicale (INSERM)**
  **75013 Paris (FR)**

- **Université de Montpellier**
  **34090 Montpellier (FR)**
- **Ecole Pratique des Hautes Etudes**
  **75014 Paris (FR)**

(72) Inventor: **GARNIER, Cyrille**
  **35137 PLEUMELEUC (FR)**

(74) Representative: **Plasseraud IP**
  **66, rue de la Chaussée d'Antin**
  **75440 Paris Cedex 09 (FR)**

Remarks:
The complete document including Reference
Table(s) and the Sequence Listing(s) can be
downloaded from the EPO website

(54) **INHIBITORY PEPTIDES FOR THE DIAGNOSTIC AND/OR TREATMENT OF TAUOPATHIES**

(57) The present invention provides inhibitory peptides for use in the diagnostic and/or treatment of tauopathies, in particular Alzheimer's Disease and Pick's Disease. The inhibitory peptides comprise a hexapeptide sequence that specifically inhibits interactions of the PHF6 sequence within pathological Tau protein.

EP 4 186 918 A1

**Description**

**Background of the Invention**

Alzheimer's disease is the most common cause of dementia, clinically characterized by the loss of cognitive function and behavorial abilities to such an extent that it interferes with a person's daily life and activities. Alzheimer's disease will affect 14 million of Europeans by 2030 with a care cost of about €140 billion/year. An estimated 6.2 million Americans age 65 and older are living with Alzheimer's today, and this number could grow to about 14 million by 2060 (Alzheimer & Dementia, 2021 Alzheimer's Disease Facts and Figures, 2021, 17(3): 327-496). The disease is associated with the accumulation of abnormal proteins in the brain: the beta-amyloid (Aβ) protein and the Tau protein. These accumulations form the two characteristic brain lesions: (i) amyloid plaques due to the accumulation of Aβ protein extracellularly (*i.e.,* outside neurons), and (ii) neurofibrillary tangles, *i.e.,* straight filaments (SFs) and paired helical filaments (PHFs), formed by the accumulation of chemically modified (abnormally phosphorylated) Tau protein intracellularly (*i.e.,* inside neurons). NFTs are not exclusive inclusions of Alzheimer's disease, as these lesions are also characteristic of other pathologies, collectively referred to as tauopathies, including progressive supranuclear palsy (PSP), Pick's disease, and several others (Hutton et al., Nature, 1998, 393: 702-705; Iseki et al., Acta Neuropathol., 2003, 105: 265-270; Querfurth et al., N. Engl. J. Med., 2010, 362: 329-344; Irwin et al., Parkinsonism Relat. Disord., 2016, 22: S29-S33; Silva *et al.,* 8. Silva et al., eLife, 2019, 8: e45457). Despite the extensive research aimed at finding approaches to halt the progression of Alzheimer's and related diseases, to date, there are no effective therapeutics able to stop or reverse the disease process and cognitive decline (Graham et al., Annu. Rev. Med., 2107, 68: 413-430; Medina et al., Int. J. Mol. Sci., 2018, 19: 1160).

Tau is now considered the critical target in treating Alzheimer's disease and other tauopathies (Congdon et al., Nature Rev. Neurol., 2018, 14: 399-415; Jadhav et al., Acta Neuropathol. Commun., 2019, 7: 22; VandeVrede et al., Neurosci. Lett., 2020, 731: 124919). Indeed, previous studies focusing on the correlation of Alzheimer's disease neuropathological changes (Aβ plaques and NFTs) with cognitive impairment have shown that the severity of cognitive impairment correlated best with the burden of abnormal Tau (Nelson et al., J. Neuropathol. Exp. Neurol., 2012, 71: 362-381). Tau is a stabilizing microtubule-associated protein that is mainly found in the axonal compartment of neurons. In the adult human brain, alternative splicing from the *MAPT* gene on chromosome 17 yields six Tau isoforms (352-441 amino acid residues; 37-46 kDa), which are distinguished by the absence or presence of one or two N-terminal inserts, and the presence of three (3R) or four (4R) microtubule-binding repeats in the C-terminal half of tau. Besides alternative splicing, Tau can undergo several posttranslational modifications such as phosphorylation, acetylation, methylation, glycation, isomerization, O-GlcNAcylation, nitration, sumoylation, glycosylation, ubiquitination, and truncation, creating a large heterogeneity of Tau molecules. Several of these posttranslational modifications impact the localization and the propensity for aggregation of tau. Previous work has shown that two six-residue segments of tau's sequence, $^{275}$VQIINK$^{280}$ (located at the beginning of Repeat 2 - SEQ ID NO: 1) and $^{306}$VQIVYK$^{311}$ (located at the beginning of Repeat 3 - SEQ ID NO: 2), drive tau's aggregation. In particular, assembly of Tau protein into paired helical filaments (PHFs) depends on $^{306}$VQIVYK$^{311}$, also termed PHF6. This motif coincides with the highest β-amyloid structure potential in tau. Point mutations in the hexapeptide region can decrease or increase aggregation, depending on the change in β-amyloid propensity (Barghorn et al., Biochem., 2004, 43: 1694-1703; von Bergen et al., J. Biol. Chem., 2001, 276: 48165-48174).

Different tau-targeting therapeutic strategies have been considered such as microtubule stabilization, immunotherapy, O-GlcNac inhibition, inhibition of kinases, Tau expression reduction and Tau aggregation inhibition (for reviews on the different tau-based strategies, see, for example: Gong et al., Drugs Aging, 2010, 27(5): 351-365; Brunden et al., Exp. Neurol., 2010, 223(2): 304-310; Boutajangout et al., Gerontology, 2014, 60(5): 381-385; Bakota et al., Drugs, 2016, 76(3): 301-313, Congdon et al., Nat. Rev. Neurol., 2018, 14(7): 399-415; Chong et al., Cell. Mol. Neurobiol., 2018, 38(5): 965-980; Hoskin et al., Expert Opin. Investig. Drugs, 2019, 28(6): 545-554; Dominguez-Meijide et al., Brain Sci., 2020, 10(11): 858). Among them, inhibition of Tau aggregation is the most widely investigated strategy in Alzheimer's disease (Jouanne et al., Eur. J. Med. Chem., 2017, 139: 153-167). Two different pharmacological strategies aiming at inhibiting Tau aggregation have been developed. One consists of the direct binding to tau, keeping it in an interaction-incompetent conformation thereby hampering its aggregation (Cisek et al., Curr. Alzheimer's Res., 2014, 11: 918-927; Seidler et al., Nature Chem., 2018, 10: 170-176). The other strategy is based on interactions that promote the stabilization of non-toxic species (Cisek et al., Curr. Alzheimer's Res., 2014, 11: 918-927; Seidler et al., Nature Chem., 2018, 10: 170-176; Uversky et al., Biochim. Biophys. Acta (BBA) Proteins Proteom., 2013, 1834: 932-951; Martinelli et al., Int. J. Mol. Sci., 2019, 20: 1322).

Although new promising therapeutic approaches targeting pathological forms of Tau are being developed and tested in clinical trials, no disease-modifying treatments of Alzheimer's disease or other tauopathies are yet available. Furthermore, a major unmet need for the development and implementation of tau-targeted therapies is precise antemortem diagnosis.

**Summary of the Invention**

The present Inventor has identified weakly to non-amyloidogenic and weakly to non-aggregative hexapeptides that are capable of efficiently inhibiting the self-associative process of the pathological Tau protein, in particular the assembly of Tau into paired helical filaments (PHFs), which depends on PHF6. More specifically, the identified hexapeptides target the interactions of the PHF6 peptide (VQIVYK, SEQ ID NO: 2) with itself and with [374]HKLTF[378] (SEQ ID NO: 3), which are observed in Alzheimer's disease and the interaction of the PHF6 peptide with [336]QVEVKS[341] (SEQ ID NO: 4), which is observed in Pick's disease - these interactions being responsible of the nucleation processes of amyloid aggregates. In particular, the present Inventors have shown that the identified hexapeptides efficiently inhibit *in vitro* PHF6 self-association and are able to interact *in silico* with both free Tau protein and Tau amyloid structures. The identified hexapeptides may be used as backbones for the development of new molecules allowing a dual diagnostic/therapeutic approach (theragnostics) for the management of tauopathies. Indeed, starting from these hexapeptides, it is possible to produce, in a single tool, molecules that can be used (i) as a therapeutic agent for the treatment of Alzheimer's disease and other tauopathies, and (ii) as a probe for the early diagnosis of tauopathies.

Consequently, the present invention provides an inhibitory peptide for use in the diagnostic and/or treatment of a tauopathy or a disorder associated with abnormal Tau aggregation in a subject, wherein the inhibitory peptide consists of, or comprises, a hexapeptide or a derivative thereof, wherein:

- the hexapeptide consists of an amino acid sequence selected from the group consisting of SEQ ID NO: 6 **(4p)**, SEQ ID NO: 18 (**4p**$_{inv}$),SEQ ID NO: 7 **(5p)**, SEQ ID NO: 19 (**5p**$_{inv}$), SEQ ID NO: 9 **(8p)**, SEQ ID NO: 21 (**8p**$_{inv}$), SEQ ID NO: 10 **(9p)**, SEQ ID NO: 22 (**9p**$_{inv}$), SEQ ID NO: 11 **(13p)**, SEQ ID NO: 23 (**13p**$_{inv}$), SEQ ID NO: 12 **(14p)**, SEQ ID NO: 24 (**14p**$_{inv}$), SEQ ID NO: 13 **(23p)**, SEQ ID NO: 25 (**23p**$_{inv}$), SEQ ID NO: 14 (**1tp**), SEQ ID NO: 26 (**1tp**$_{inv}$), SEQ ID NO: 15 **(4tp)**, SEQ ID NO: 27 **(4tpinv)**, SEQ ID NO: 16 **(6tp)**, and SEQ ID NO: 28 **(6tpinv)**, and
- the hexapeptide derivative consists of an amino acid sequence selected from said group, wherein the amino acid sequence comprises a single conservative amino acid substitution, or wherein the amino acid sequence comprises conservative amino acid substitutions at two of positions 1, 3 and 5 or at two of positions 2, 4 and 6.

In certain embodiments, the inhibitory peptide for use in the diagnostic and/or treatment of a tauopathy or a disorder associated with abnormal Tau aggregation in a subject, consists of, or comprises, a hexapeptide or a derivative thereof, wherein:

- the hexapeptide consists of an amino acid sequence selected from the group consisting of SEQ ID NO: 6 **(4p)**, SEQ ID NO: 18 (**4p**$_{inv}$),SEQ ID NO: 7 **(5p)**, SEQ ID NO: 19 (**5p**$_{inv}$), SEQ ID NO: 14 (**1tp**), SEQ ID NO: 26 (**1tp**$_{inv}$), SEQ ID NO: 15 **(4tp)**, SEQ ID NO: 27 **(4tpinv)**, SEQ ID NO: 16 **(6tp)**, and SEQ ID NO: 28 **(6tpinv)**, and
- the hexapeptide derivative consists of an amino acid sequence selected from said group, wherein the amino acid sequence comprises a single conservative amino acid substitution, or wherein the amino acid sequence comprises conservative amino acid substitutions at two of positions 1, 3 and 5 or at two of positions 2, 4 and 6.

In certain embodiments, the inhibitory peptide for use in the diagnostic and/or treatment of a tauopathy or a disorder associated with abnormal Tau aggregation in a subject, consists of, or comprises, a hexapeptide or a derivative thereof, wherein:

- the hexapeptide consists of an amino acid sequence selected from the group consisting of SEQ ID NO: 6 **(4p)**, SEQ ID NO: 7 **(5p)**, SEQ ID NO: 14 (**1tp**), SEQ ID NO: 15 **(4tp)**, and SEQ ID NO: 16 **(6tp)**, and
- the hexapeptide derivative consists of an amino acid sequence selected from said group, wherein the amino acid sequence comprises a single conservative amino acid substitution, or wherein the amino acid sequence comprises conservative amino acid substitutions at two of positions 1, 3 and 5 or at two of positions 2, 4 and 6.

In certain embodiments, the inhibitory peptide for use in the diagnostic and/or treatment of a tauopathy or a disorder associated with abnormal Tau aggregation in a subject, consists of, or comprises, a hexapeptide or a derivative thereof, wherein:

- the hexapeptide consists of an amino acid sequence selected from the group consisting of SEQ ID NO: 14 (**1tp**), and SEQ ID NO: 16 **(6tp)**, and
- the hexapeptide derivative consists of an amino acid sequence selected from said group, wherein the amino acid sequence comprises a single conservative amino acid substitution, or wherein the amino acid sequence comprises conservative amino acid substitutions at two of positions 1, 3 and 5 or at two of positions 2, 4 and 6.

In certain embodiments, the hexapeptide or hexapeptide derivative within an inhibitory peptide is linked to a heterologous moiety selected from the group consisting of detectable moieties, cell penetrating agents, stability enhancing moieties, tags allowing cellular degradation, and blood-brain barrier shuttle moieties.

In other embodiments, the hexapeptide or hexapeptide derivative within an inhibitory peptide is linked to a detectable moiety and to a heterologous moiety selected from the group consisting of cell penetrating agents, tags allowing cellular degradation, stability enhancing moieties, and blood-brain barrier shuttle moieties.

In certain embodiments, the detectable moiety is suitable for detection by positron emission tomography (PET), in particular may be to a radionuclide selected from the group consisting of $^{11}C$, $^{13}N$, $^{15}O$, $^{18}F$, $^{62}Cu$, $^{64}Cu$, $^{67}Cu$, $^{67}Ga$, $^{68}Ga$, $^{44}Sc$, $^{43}Sc$, $^{47}Sc$, $^{124}I$, $^{76}Br$, and $^{82}Rb$.

In some embodiments, the hexapeptide or hexapeptide derivative is linked to a detectable moiety, and the detectable moiety is a multimodal nanoparticle.

In certain embodiments, the hexpeptide or hexapeptide derivative is cyclized.

The present invention further provides a combination of at least two inhibitory peptides as defined above for use in the diagnosis and/or treatment of a tauopathy or a disorder associated with abnormal Tau aggregation in a subject.

The present invention also provides a pharmaceutical composition comprising at least one inhibitory peptide as defined above and at least one pharmaceutically acceptable carrier or excipient for use in the diagnosis and/or treatment of a tauopathy or a disorder associated with abnormal Tau aggregation in a subject.

In certain embodiments, the pharmaceutical composition further comprises at least one additional biologically active agent.

In certain embodiments, a diagnosis according to the present invention is a diagnosis of an early stage of a tauopathy.

In certain embodiments, a diagnosis according to the present invention is used to monitor the effectiveness of a tauopathy treatment in a patient.

In certain embodiments, the tauopathy is selected from the group consisting of Alzheimer's disease (AD), Pick's disease (PD), progressive supranuclear palsy (PSP), corticobasal degeneration (CBD), argyrophilic grain disease (AGD), globular glial tauopathy (GGT), aging-related Tau astrogliopathy (ARTAG), frontotemporal dementia and parkinsonism linked to chromosome 17 (FTDP-17), subacute sclerosing panencephalitis (SSPE), Guadeloupean parkinsonism, Western pacific amyotrophic lateral sclerosis and parkinsonism-dementia complex (ALS/PDC), chronic traumatic encephalopathy (CTE), Huntington Disease (HD); Niemann-Pick disease type C (NPC); Down syndrome; post-encephalitic parkinsonism (PEP), and myotonic dystrophies (types 1 and 2), in particular Alzheimer's disease (AD) and Pick's disease (PD).

These and other objects, advantages and features of the present invention will become apparent to those of ordinary skill in the art having read the following detailed description of the preferred embodiments.

**Brief Description of the Drawing**

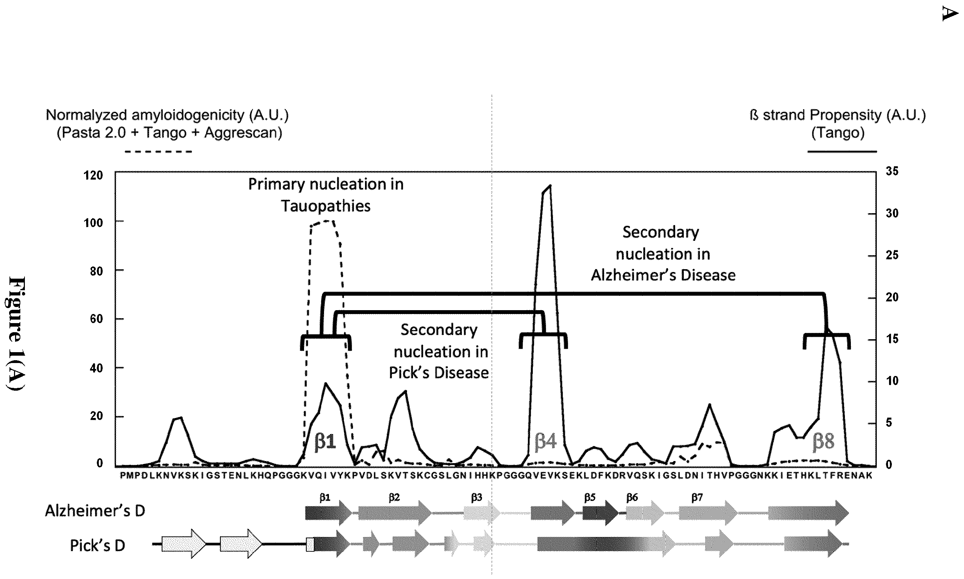

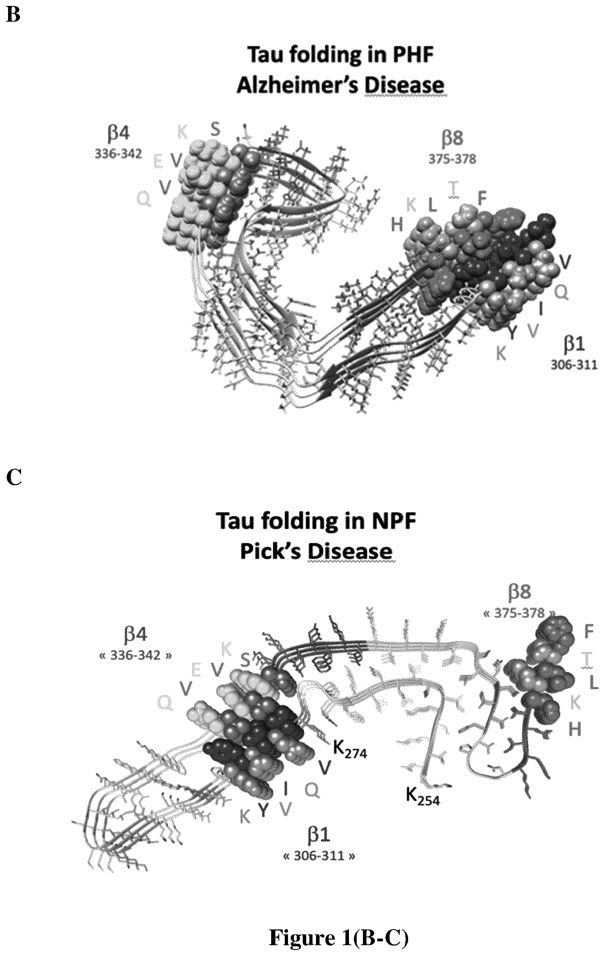

**Figure 1**. Sequences analyses by predictive methods and domains involved in filaments nucleation. PHF6 (306-311 "VQIVYK", β1 in PHF's structure) shows strong amyloidogenic properties (blue curve). Secondary structure prediction shows that the whole sequence tends to fold as β-strand (red curve). B-Tau folding in PHF (Alzheimer's disease) and NPF (Pick's disease) determined by cryo-EM (see Examples section). In both structures, Tau proteins stack on top of each other perpendicularly to the fiber axis through parallel β-sheet interactions. Tau protein folds as a hairpin stabilized by intramolecular interactions corresponding to an inter-digitation of amino acids side chains (domains R3 and R4 in Alzheimer's disease to which are added the end of R1 in Pick's disease). Peptides involved in filaments primary and secondary nucleations phases are represented as spheres. Primary nucleation is the result of $^{301}VQIVYK^{306}$ (dark/light blue) self-assembly as a parallel β-sheet. Secondary nucleation corresponds to $^{301}VQIVYK^{306}$ amino acids side chain inter-digitation with $^{374}HKLTF^{378}$ (red/orange) in Alzheimer's disease and with $^{336}QVEVKS^{341}$ (dark/light green) in Pick disease.

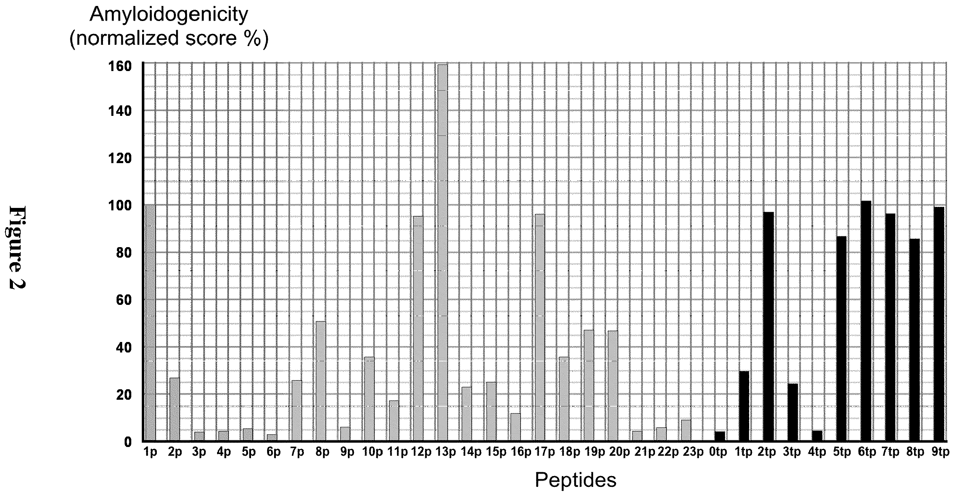

**Figure 2.** Prediction, by bioinformatics means, of the amyloidogenic character of the 22 first hexapeptides (in red) and 10 additional hexapeptides (in green) compared to the PHF6 peptide (**1p**). The reference peptide **1p** is considered to be 100% amyloidogenic, the score of the other peptides is normalized as function of 1p.

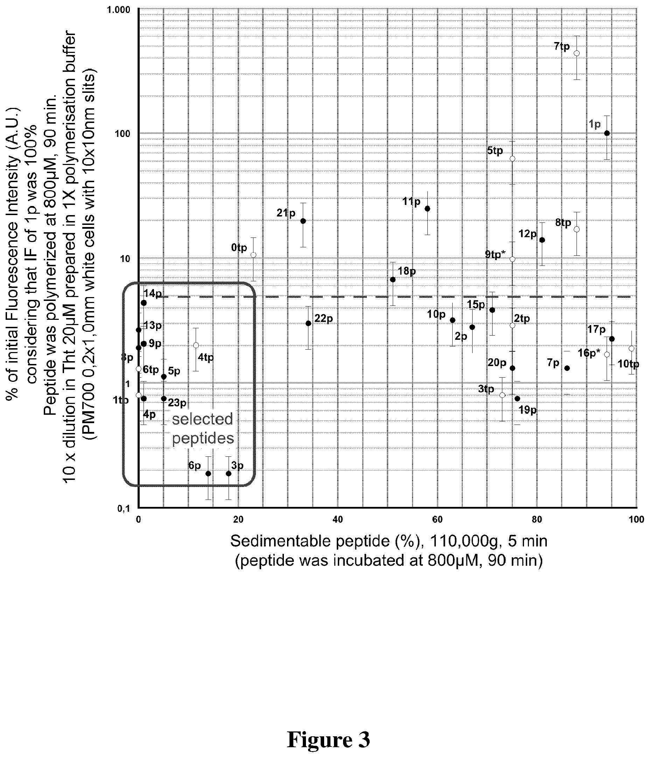

**Figure 3.** Garnier-Delamarche representation of the 22 first hexapeptides (in red) and 10 additional hexapeptides (in green). The peptides fluorescence score was normalized as function of 1p fluorescence score (internal control). The **16p** and **9tp** peptide (in blue) could not be tested at 800 μM like the other peptides due to their low solubility in aqueous medium. The % of sedimentation and of fluorescence were determined at a concentration of 288 μM.

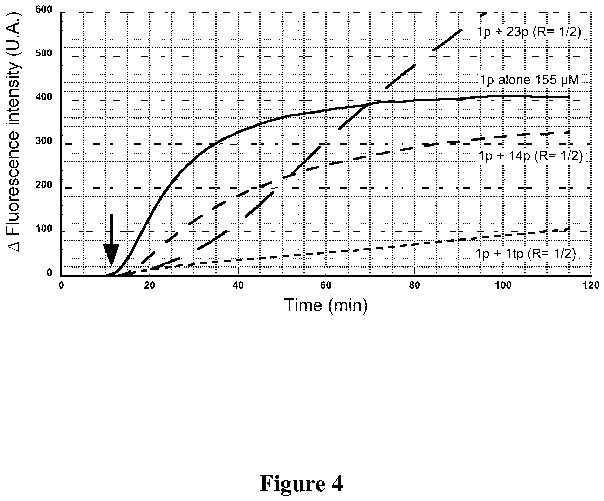

**Figure 4.** Typical polymerization kinetics of **1p** in the presence or absence of selected peptides. **1p** alone (red); **1p** + **4p** (blue); **1p** + **23p** (green); **1p** + **1tp** (orange). The arrow indicates the time at which the fibrillation buffer 5X was added, [ThT] = 32 mM; PM = 800 V; slits 03 nm.

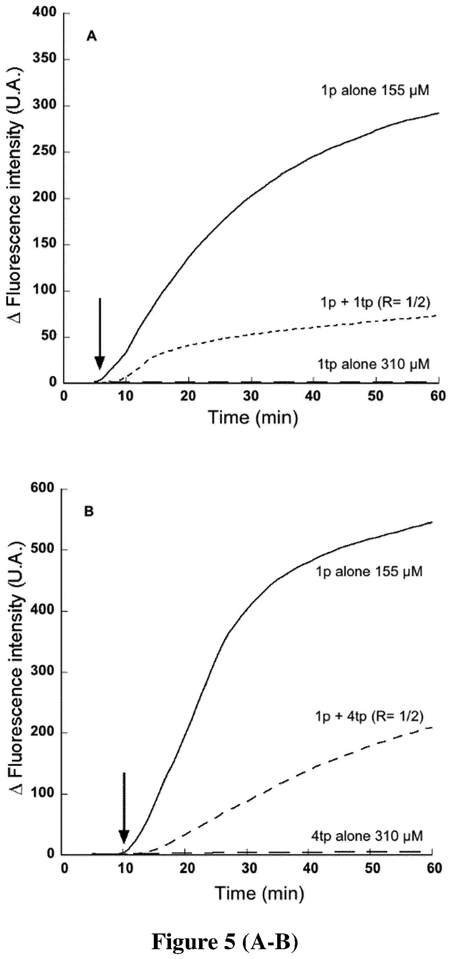

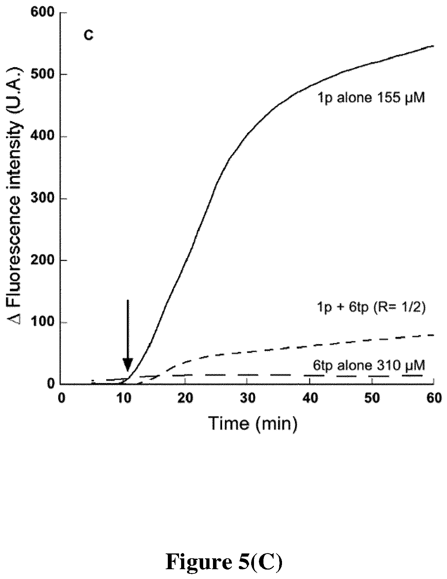

**Figure 5.** Polymerization kinetics of **1p** in the presence or absence of selected peptides. (A) kinetics of **1p** alone (red), **1p** + **1tp** (green), and **1tp** alone (blue); **(B)** kinetics of **1p** alone (red), **1p** + **4tp** (green), and **4tp** alone (blue); (C) kinetics of 1p alone (red), **1p** + **6tp** (green), and **6tp** alone (blue). [ThT] = 32 $\mu$M, MW = 800 V, slits 03 nm, [1p] = 155 $\mu$M, [selected peptide] = 310 $\mu$M. The arrow indicates the time at which the fibrillation buffer 5X was added.

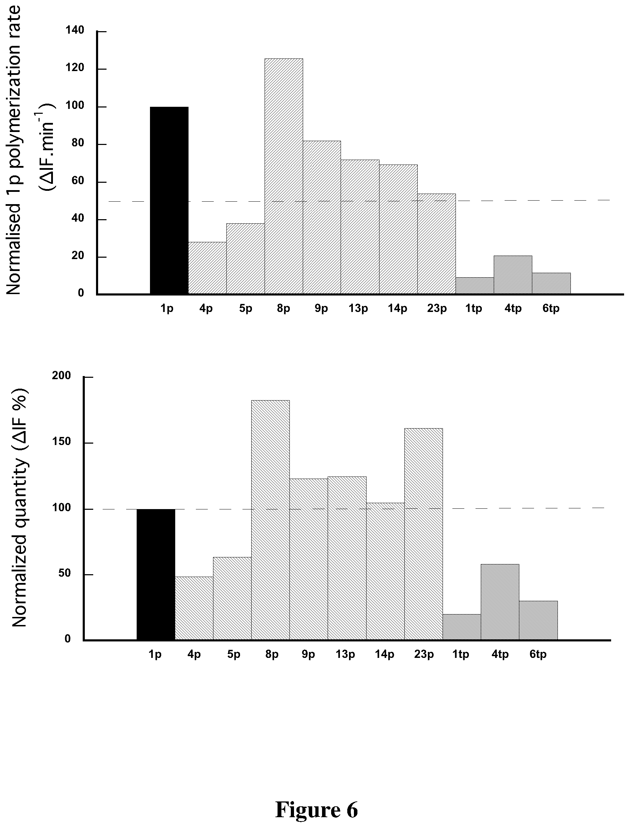

Figure 6. Results of fluorescence intensity and maximum rate of polymerization kinetics of **1p** in the presence of selected peptides. The maximum polymerization rate is shown on the left; the fluorescence intensity at 100 minutes is shown on the right. All the results were normalized by considering the fluorescence intensity at 100 minutes and the maximum polymerization rate of **1p** as 100%. Thresholds plotted at 50% for rate 100% for fluorescence intensity highlight the peptides of greatest interest. For each analysis, [ThT] = 32 $\mu$M, MW = 800 V, slits 03 nm; **[1p]** = 155 $\mu$M, [selected peptide] = 310 $\mu$M.

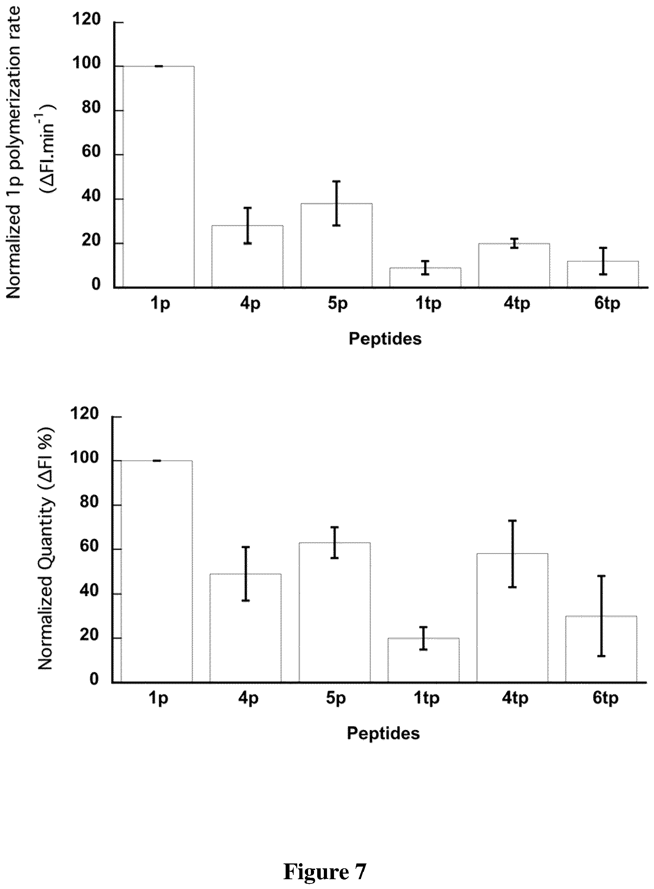

**Figure 7.** Results of fluorescence intensity and maximum rate of polymerization kinetics of **1p** in the presence of the peptides with the most interesting inhibitory properties. The maximum polymerization rate is shown on the left; the fluorescence intensity at 100 minutes is shown on the right. All the results were normalized by considering the fluorescence intensity at 100 minutes and the maximum polymerization rate of **1p** as 100%. For each analysis, [ThT] = 32 $\mu$M, MW = 800 V, slits 03 nm; **[1p]** = 155 $\mu$M, [selected peptide] = 310 $\mu$M.

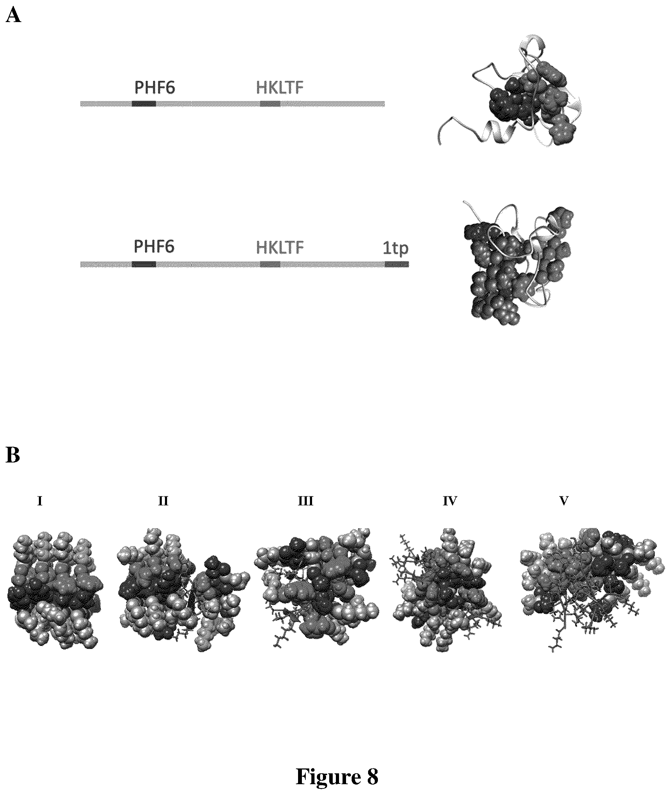

**Figure 8.** Effect of **6tp** (purple) on *in silico* simulated PHF's core structure. [301]VQIVYK[306] (dark/light blue) self-assembles as a parallel $\beta$-sheet and interacts with [374]HKLTF[378] (red/orange) by amino acids side chain inter-digitation. The number of **6tp** peptides tested varies from 1 (stoichiometry 0.25; panel II) to 4 (stoichiometry 1, panel V). The [301]VQIVYK[306] / [374]HKLTF[378] structure is affected from the stoichiometry of 0.25 and is totally destabilized at the stoichiometry of 0.5.

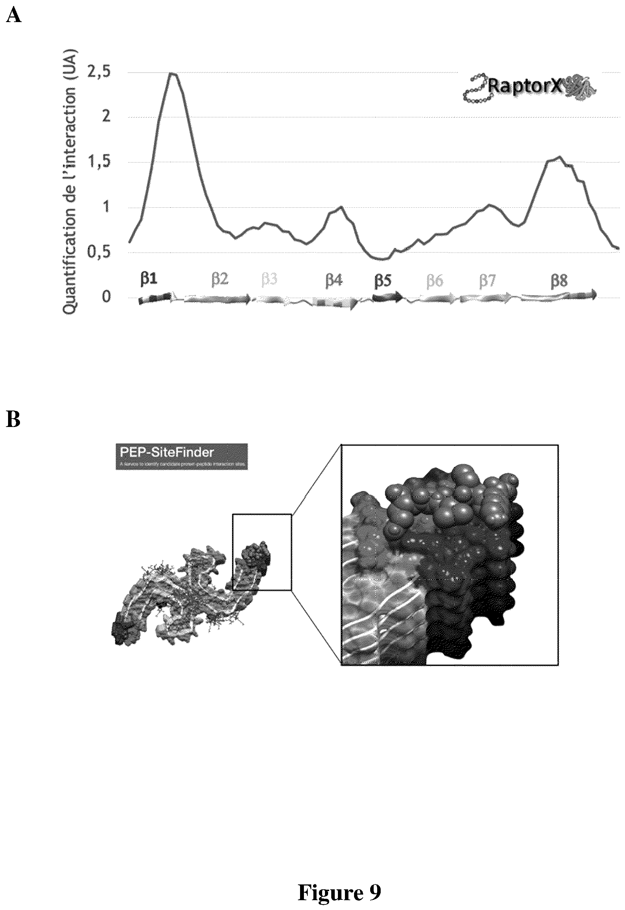

**Figure 9.** *In silico* prediction of the interaction of 4tp with free Tau protein (R3/R4 repeats) **(A)** and with PHF structures **(B).**

## Definitions

Throughout the specification, several terms are employed that are defined in the following paragraphs.

As used herein, the term *"subject"* refers to a human or another mammal (*e.g.*, primate, mouse, rat, rabbit, dog, cat, horse, cow, goat, pig, camel, and the like), including laboratory animals, that may or may not have a disease or disorder, in particular a tauopathy. Non-human subjects may be transgenic or otherwise modified animals. In many embodiments of the present invention, the subject is a human being. In such embodiments, the subject is often referred to as an *"individual"* or a *"patient"*. The terms "individual" and "patient" do not denote a particular age. The term "patient" more specifically refers to an individual suffering from a disease or disorder, in particular a tauopathy.

The terms *"tau"* and *"Tau protein"* are used herein interchangeably. They refer to the human protein encoded by a single gene, *MAPT* (microtubule-associated protein tau), which maps to chromosome 17q21.31. The terms include any one of the six highly soluble protein isoforms produced in the adult human brain by alternative splicing. The terms *"abnormal tau"*, *"abnormal form(s) of tau"*, *"pathological tau"*, and *"pathological form(s) of tau"* are used herein interchangeably. They refer to any non-functional or deregulated form of tau. Abnormal posttranslational modifications are believed to be one of the main causes of this failure. Abnormal phosphorylation (hyperphosphorylation), acetylation, glycation, ubiquitination, nitration, proteolytic cleavage (truncation), conformation changes, and some other modifications have been proposed to cause the loss of normal function and the gain of pathological features of Tau protein. The loss of function of deregulation of the Tau protein results in its withdrawal from the microtubule to which it is normally linked. Genetic, pathological, and biochemical analyses have proved that abnormal Tau protein plays a major role in the pathogenesis of all tauopathies.

As used herein, the term *"tauopathy"* has its art-understood meaning and refers to a group of clinically, biochemically, and morphologically heterogeneous neurodegenerative diseases characterized by the deposition of abnormal tau. Several lines of evidence suggest that Tau aggregation is central to the neurodegenerative process in tauopathies. In tauopathies, soluble Tau disconnects from microtubules and forms abnormal, aggregated filamentous assemblies of hyperphosphorylated tau. Based on the distinct involvement of anatomic areas, cell types, and presence of distinct isoforms of Tau in pathological deposits, several neuropathologic phenotypes are distinguished. The term *"primary tauopathy"* refers to disorders in which Tau protein deposition is the predominant feature. The term *"secondary*

*tauopathy"* refers to Tau pathologies considered as having another and diverse driving force (*e.g.*, amyloid, ageing, trauma, genetics, infectious diseases, toxins, autoimmune diseases, and other unknown factors). The nomenclature of primary tauopathies overlaps with the modern classification of frontotemporal lobar degeneration (FTLD). In the adult human brain, six Tau isoforms are expressed that differ by the presence or absence of one or two N-terminal inserts and of the second of four semiconserved repeats. As a consequence, half of the Tau isoforms have three repeats (3R tau), whereas the other half of the isoforms have four repeats (4R tau). Tauopathies can be characterized based on the isoform composition of their filaments, as in tauopathies, the ratio of 3R tau/4R Tau differs and is dependent on the disorder. Thus, tauopathies have been classified in different groups: *"3R tauopathies"* where 3R Tau is predominant, *"4R tauopathies"* where 4R is predominant, and *"mixed 3R/4R tauopathies"* where the ratio of three repeat (3R) to four repeat (4R) Tau is approximately equal. Examples of 3R-tauopathies include Pick's disease (PiD), also called frontotemporal dementia (FTD). Examples of primary 4R-tauopathies include progressive supranuclear palsy (PSP), corticobasal degeneration (CBD), argyrophilic grain disease (AGD), globular glial tauopathy (GGT), and aging-related Tau astrogliopathy (ARTAG). Examples of primary mixed 3R/4R tauopathies include frontotemporal dementia and parkinsonism linked to chromosome 17 (FTDP-17); subacute sclerosing panencephalitis (SSPE), also known as Dawson disease. Other examples of primary mixed 3R/4R tauopathies include the geographically isolated tauopathies such as Guadeloupean parkinsonism, Western pacific amyotrophic lateral sclerosis and parkinsonism-dementia complex (ALS/PDC), and Nodding syndrome of northern Uganda. Examples of secondary mixed 3R/4R tauopathies include Alzheimer's disease (AD), a tauopathy that is secondary to amyloid deposition; chronic traumatic encephalopathy (CTE), also known as dementia pugilistica, which is secondary to trauma; Huntington Disease (HD); Niemann-Pick disease type C (NPC); Down syndrome; and post-encephalitic parkinsonism (PEP). Other secondary tauopathies include myotonic dystrophies (types 1 and 2).

As used herein, the term *"disorder associated with abnormal Tau aggregation"* encompasses any disease or clinical condition which is not defined as a tauopathy but is a disorder wherein abnormal Tau deposition is present. Such disorders include, but are not limited to: Lewy body disease (LBD), Creutzfeldt-Jacob disease, Gerstmann-Straussler-Scheinker disease, inclusion-body myositis, prion protein cerebral amyloid angiopathy, amyotrophic lateral sclerosis, non-Guamanian motor neuron disease with neurofibrillary tangles, diffuse neurofibrillary tangles with calcification, Hallervorden-Spatz disease, multiple system atrophy, pallido-ponto-nigral degeneration, Tau panencephalopathy, AD-like with astrocytes, certain prion diseases (GSS with Tau), mutations in LRRK2, familial British dementia, familial Danish dementia, neurodegeneration with brain iron accumulation, SLC9A6-related mental retardation, white matter tauopathy with globular glial inclusions, traumatic stress syndrome, epilepsy, hereditary cerebral hemorrhage with amyloidosis (Dutch type), mild cognitive impairment (MCI), multiple sclerosis, Parkinson's disease, atypical parkinsonism, HIV related dementia, adult onset diabetes, senile cardiac amyloidosis, endocrine tumors, glaucoma, ocular amyloidosis, primary retinal degeneration, macular degeneration (such as age-related macular degeneration (AMD)), optic nerve drusen, optic neuropathy, optic neuritis, and lattice dystrophy.

As used herein, the term *"inhibit"* means to prevent something from happening, to delay occurrence of something happening, and/or to reduce the extent or likelihood of something happening. Thus, the terms "inhibiting abnormal Tau aggregation" and "inhibiting the formation of abnormal Tau aggregates", which are used herein interchangeably, are intended to encompass preventing, delaying, and/or reducing the likelihood of occurrence of abnormal Tau aggregation as well as reducing the extent of abnormal Tau aggregates.

The term *"treatment"* is used herein to characterize a method or process that is aimed at (1) delaying or preventing the onset of a disease, disorder or condition (here a tauopathy); (2) slowing down or stopping the progression, aggravation or deterioration of the disease, disorder or condition; (3) bringing about amelioration of the symptoms of the disease, disorder or condition; or (4) curing the disease, disorder or condition. A treatment may be administered after initiation of the disease, disorder or condition, for a therapeutic action. Alternatively, a treatment may be administered prior to the onset of the disease, disorder or condition, for a prophylactic or preventive action. In this case, the term *"prevention"* is used.

The term *"diagnosis"*, as used herein, includes the assessment of a subject's susceptibility to a disease (here a tauopathy), determination as to whether a subject presently has the disease, and also the prognosis of a subject affected by the disease. As will be understood by one skilled in the art, such assessment normally may not be correct for 100% of subjects to be diagnosed, although it preferably is correct. The term, however, requires that a statistically significant part of the subjects can be identified as suffering from the disease or having a predisposition thereto. If a part is statistically significant can be determined simply by a person skilled in the art using several well-known statistical evaluation tools, for example, determination of confidence intervals, determination of p values, Student's t-test, Mann-Whitney test, etc. Details are provided in Dowdy and Wearden, Statistics for Research, John Wiley & Sons, New York 1983. The preferred confidence intervals are at least 50%, at least 60%, at least 70%, at least 80%, at least 90%), at least 95%. The p values are preferably 0.2, 0.1 or 0.05. The terms *"susceptibility"*, *"propensity"* and *"predisposition"* are used herein interchangeably, and refer to a higher than normal probability of developing a disease (here a tauopathy).

The terms *"labeled"* and *"labeled with a detectable agent or moiety"* are used herein interchangeably to specify that an entity (*e.g.,* an inhibitory peptide of the invention) can be visualized, for example following binding to another entity (*e.g.,* the PHF6 peptide or to an abnormal Tau protein). Preferably, the detection agent or moiety is selected such that it generates a signal which can be measured and whose intensity is related to (*e.g.,* proportional to) the amount of bound entity. A wide variety of systems for labeling and/or detecting peptides are well-known in the art. Labeled peptides can be prepared by incorporation of, or conjugation to, a label that is directly or indirectly detectable by spectroscopy, photochemical, biochemical, immunochemical, electrical, optical, chemical or other means. Suitable detectable agents include, but are not limited to, radionuclides, fluorophores, chemiluminescent agents, microparticles, enzymes, colorimetric labels, magnetic labels, haptens, Molecular Beacons, and aptamer beacons.

A *"pharmaceutical composition"* is defined herein as comprising an effective amount of at least one inhibitory peptide described herein, and at least one pharmaceutically acceptable carrier or excipient.

As used herein, the term *"effective amount"* refers to any amount of a compound *(e.g.,* an inhibitory peptide), agent, or composition that is sufficient to fulfil its intended purpose(s), *e.g.,* a desired biological, diagnostic, or medicinal response in a cell, tissue, system or subject. An effective amount of an inhibitory peptide is an amount that can elicit a measurable amount of a desirable outcome, e.g., inhibition of abnormal Tau aggregation or cytotoxicity; for a diagnostic assay, an amount that can detect a target of interest; or in a method of treatment, an amount that can reduce or ameliorate by a measurable amount, a symptom of the disease or condition that is being treated.

As used herein, the term *"pharmaceutically acceptable carrier or excipient"* refers to a carrier medium which does not interfere with the effectiveness of the biological activity of the active ingredient(s) and which is not excessively toxic to the host at the concentration at which it is administered. The term includes solvents, dispersion media, coatings, antibacterial and antifungal agents, isotonic agents, adsorption delaying agents, and the like. The use of such media and agents for pharmaceutically active substances is well known in the art (see for example *"Remington's Pharmaceutical Sciences"*, E.W. Martin, 18th Ed., 1990, Mack Publishing Co.: Easton, PA, which is incorporated herein by reference in its entirety). Preferably, the pharmaceutical carrier or excipient is acceptable in human medicine. In certain embodiments, the pharmaceutically acceptable carrier or excipient is a veterinary acceptable carrier or excipient.

The terms *"approximately"* and *"about"*, as used herein in reference to a number, generally include numbers that fall within a range of 10% in either direction of the number (greater than or less than the number) unless otherwise stated or otherwise evident from the context (*e.g.,* where such number would exceed 100% of a possible value).

**Detailed Description of Certain Preferred Embodiments**

As mentioned above, the present invention provides hexapeptide sequences and inhibitory peptides for their use in both the diagnosis and the treatment of tauopathies and disorders associated with abnormal Tau aggregation.

**I - Inhibitory Peptides, Hexapeptides and Hexapeptide-Based Molecules**

When Tau is functional, it is linked to a microtubule through its repeat domains (R1, R2, R3 and R4). PHF6 ($^{306}$VQIVYK$^{311}$, SEQ ID NO: 2), being the integral part of the R3 domain, it is not accessible under normal conditions *(i.e.,* in the absence of a disease or pre-disease state). The loss of function or deregulation of the Tau protein (phosphorylation or other...) and therefore its withdrawal from the microtubule is recognized as a phenomenon responsible for the onset of tauopathy. As the abnormal Tau protein is found free in the cytoplasm, the PHF6 peptide then becomes accessible to inhibitory peptides described herein.

The term *"inhibitory peptide"*, as used herein, refers to a molecule that consists of, or comprises, a hexapeptide, or hexapeptide derivative, which reduces, inhibits, or prevents the self-association process of the abnormal Tau protein, in particular abnormal Tau's self-association process that involves the PHF6 peptide. In certain embodiments, the term "inhibitory peptide" refers to a molecule consisting of, or comprising, a hexapeptide, or hexapeptide derivative, that reduces, inhibits, or prevents the interactions of the PHF6 peptide within the abnormal Tau protein. The term "inhibitory peptide" also refers to a molecule consisting of, or comprising, a hexapeptide, or hexapeptide derivative, that reduces, inhibits, or prevents the self-association process of the PHF6 peptide *in vitro.* Hexapeptides and hexapeptide derivatives provided herein are L-hexapeptides, *i.e.,* peptides consisting of six L-amino acid residues. The phrase *"an inhibitory peptide comprising a hexapeptide"* means that the inhibitory peptide is constituted by the amino acid sequence of the hexapeptide, and the hexapeptide is lengthened by a few amino acids at one end or at both of its ends. For example, the hexapeptide may be lengthened by a total of 1, 2, 3, 4, 5, 6, 7, or 8 amino acid residues. These amino acid residues are such that they do not modify the inhibiting properties of the hexapeptide.

**1. Sequences of the L-Hexapeptides**

A L-hexapeptide according to the present invention consists of one of the amino acid sequences presented below in Table 1.

**Table 1.** Sequences of L-hexapeptides.

| Name | Sequence | SEQ ID NO: | Name | Sequence | SEQ ID NO: |
|------|----------|-----------|------|----------|-----------|
| **3p** | VKYQVI | 5 | **3p$_{inv}$** | IVQYKV | 17 |
| **4p** | VQKIYV | 6 | **4p$_{inv}$** | VYIKQV | 18 |
| **5p** | IKQVYV | 7 | **5p$_{inv}$** | VYVQKI | 19 |
| **6p** | VVQKYI | 8 | **6p$_{inv}$** | IYKQVV | 20 |
| **8p** | QKYIVV | 9 | **8p$_{inv}$** | VVIYKQ | 21 |
| **9p** | IYVQKV | 10 | **9p$_{inv}$** | VKQVYI | 22 |
| **13p** | YIVVQK | 11 | **13p$_{inv}$** | KQVVIY | 23 |
| **14p** | KVQYVI | 12 | **14p$_{inv}$** | IVYQVK | 24 |
| **23p** | QVIKYV | 13 | **23p$_{inv}$** | VYKIVQ | 25 |
| **1tp** | VKIVYQ | 14 | **1tp$_{inv}$** | QYVIKV | 26 |
| **4tp** | VKIQYV | 15 | **4tp$_{inv}$** | VYQIKV | 27 |
| **6tp** | YQIVVK | 16 | **6tpinv** | KVVIQY | 28 |

Thus, an inhibitory peptide disclosed herein consists of, or comprises, a hexapeptide having an amino acid sequence selected from the group consisting of SEQ ID NOs: 5-28. In other words, an inhibitory peptide described herein consists of, or comprises, any one of hexapeptides **3p** (SEQ ID NO: 5), **3pinv** (SEQ ID NO: 17), **4p** (SEQ ID NO: 6), **4pinv** (SEQ ID NO: 18), **5p** (SEQ ID NO: 7), **5p$_{inv}$** (SEQ ID NO: 19), **6p** (SEQ ID NO: 8), **6pinv** (SEQ ID NO: 20), **8p** (SEQ ID NO: 9), **8pinv** (SEQ ID NO: 21), **9p** (SEQ ID NO: 10), **9pinv** (SEQ ID NO: 22), **13p** (SEQ ID NO: 11), **13pinv** (SEQ ID NO: 23), **14p** (SEQ ID NO: 12), **14pinv** (SEQ ID NO: 24), **23p** (SEQ ID NO: 13), **23pinv** (SEQ ID NO: 25), **1tp** (SEQ ID NO: 14), **1tp$_{inv}$** (SEQ ID NO: 26), **4tp** (SEQ ID NO: 15), **4tpinv** (SEQ ID NO: 27), **6tp** (SEQ ID NO: 16), and **6tpinv** (SEQ ID NO: 28).

However, in preferred embodiments, an inhibitory peptide disclosed herein consists of, or comprises, a hexapeptide identified, by the present Inventors, as being weakly or non-amyloidogenic (compared to the PFH6 peptide) and/or weakly or non-aggregative. The term *"weakly or non-amyloid"*, when used herein to characterize a hexapeptide, refers to a hexapeptide that does not form, or does not tend to, form fibrils, in particular in comparison to the PHF6 peptide. The term *"weakly or non-aggregative"*, when used herein to characterize a hexapeptide, refers to a hexapeptide that does not, or does not tend to, form self-aggregates, in particular in comparison to the PHF6 peptide. Thus, in preferred embodiments, an inhibitory peptide described herein consists of, or comprises, any one of hexapeptides **4p** (SEQ ID NO: 6), **4pinv** (SEQ ID NO: 18), **5p** (SEQ ID NO: 7), **5p$_{inv}$** (SEQ ID NO: 19), **8p** (SEQ ID NO: 9), **8pinv** (SEQ ID NO: 21), **9p** (SEQ ID NO: 10), **9pinv** (SEQ ID NO: 22), **13p** (SEQ ID NO: 11), **13pinv** (SEQ ID NO: 23), **14p** (SEQ ID NO: 12), **14p$_{inv}$** (SEQ ID NO: 24), **23p** (SEQ ID NO: 13), **23pinv** (SEQ ID NO: 25), **1tp** (SEQ ID NO: 14), **1tp$_{inv}$** (SEQ ID NO: 26), **4tp** (SEQ ID NO: 15), **4tpinv** (SEQ ID NO: 27), **6tp** (SEQ ID NO: 16), and **6tpinv** (SEQ ID NO: 28). In particular, the inhibitory peptide may consist of, or comprise, any one of hexapeptides **4p, 5p, 8p, 9p, 13p, 14p, 23p, 1tp**, **4tp,** and **6tp.**

In certain embodiments, an inhibitory peptide described herein consists of, or comprises, any one of hexapeptides **4p** (SEQ ID NO: 6), **4pinv** (SEQ ID NO: 18), **5p** (SEQ ID NO: 7), **5p$_{inv}$** (SEQ ID NO: 19), **8p** (SEQ ID NO: 9), **8pinv** (SEQ ID NO: 21), **9p** (SEQ ID NO: 10), **13p** (SEQ ID NO: 11), **14p** (SEQ ID NO: 12), **14p$_{inv}$** (SEQ ID NO: 24), **23pinv** (SEQ ID NO: 25), **1tp** (SEQ ID NO: 14), **1tp$_{inv}$** (SEQ ID NO: 26), **4tp** (SEQ ID NO: 15), **4tpinv** (SEQ ID NO: 27), and **6tpinv** (SEQ ID NO: 28).

In certain preferred embodiments, an inhibitory peptide for use in a method of diagnosis and/or treatment of tauopathies according to the present invention consists of, or comprises, a hexapeptide identified, by the present Inventors, as efficiently inhibiting the self-associative process of the PHF6 peptide *in vitro.* Thus, in certain preferred embodiments, an inhibitory peptide for use in a method according to the present invention consists of, or comprises, any one of hexapeptides **4p** (SEQ ID NO: 6), **4pinv** (SEQ ID NO: 18), **5p** (SEQ ID NO: 7), **5pinv** (SEQ ID NO: 19), **1tp** (SEQ ID NO: 14), **1tp$_{inv}$** (SEQ ID NO: 26), **4tp** (SEQ ID NO: 15), **4tpinv** (SEQ ID NO: 27), **6tp** (SEQ ID NO: 16), and **6tpinv** (SEQ ID NO: 28). In particular, the inhibitory peptide may consist of, or comprise, any one of hexapeptides **4p, 5p, 1tp**, **4tp,** and **6tp.** In certain embodiments, the inhibitory peptide consists of, or comprises, any one of hexapeptides **1tp** and **6tp.**

In certain embodiments, the hexapeptide is modified to include modifications of termini such as acetylation and amidation. Thus, the C-terminus of a hexapeptide described herein may be amidated and/or the N-terminus thereof may be acetylated. For example, when hexapeptide **3p** (SEQ ID NO: 5) is N-acetylated and C-amidated, it is written as Ac-VKYQVI-NH2.

In certain embodiments, the hexapeptide is in a salt form well-known in the art, in particular pharmaceutically acceptable salts. As used herein, the term *"pharmaceutically acceptable salt"* refers to salts of a hexapeptide that retain the biological activity of the parent hexapeptide, and which are not biologically or otherwise desirable. Examples of pharmaceutically acceptable salts include, but are not limited to, a hydrochloride salt, an acetate salt, a TFA salt, and a sodium chloride salt.

**2. L-Hexapeptide Derivatives**

The present invention also provides L-hexapeptide derivatives. In certain embodiments, a L-hexapeptide derivative according to the present invention consists of one of the amino acid sequences presented in Table 1, wherein the amino acid sequence comprises a single conservative amino acid substitution. In other embodiments, a L-hexapeptide derivative according to the present invention consists of one of the amino acid sequences presented in Table 1, wherein the amino acid sequence comprises conservative amino acid substitutions at two of positions 1, 3 and 5 or at two of positions 2, 4 and 6.

As used herein, the term *"conservative amino acid substitution"* refers to the replacement of an amino acid by another, without altering the overall conformation and function of the hexapeptide, and refers more specifically to the replacement of an amino acid residue with one having similar properties (such as, for example, polarity, hydrogen bonding potential, acidic, basic, shape, hydrophobic, aromatic, and the like). The meaning of a conservative amino acid substitution is well known in the art, as are amino acids with similar properties. For example, in the context of the present invention, Valine (V), a hydrophobic aliphatic amino acid, may be replaced with Alanine (A), Leucine (L) or isoleucine (I); Lysine (K), a basic amino acid, may be replaced with Arginine (R) or Histidine (H), in particular with Arginine (R); Isoleucine (I), a hydrophobic aliphatic amino acid, may be replaced with Valine (V), Leucine (L) or Alanine (A); Glutamine (Q), a neutral polar amino acid, may be replaced with Asparagine (N), Serine (S), or Threonine (T), in particular with Asparagine (N); and Tyrosine (Y), an aromatic amino acid, may be replaced with Tryptophan (W) or Phenylalanine (F).

As used herein, the terms *"position 1, 2, 3, 4, 5 or 6"* refers to the first, second, third, fourth, fifth or sixth location of an amino acid residue in an hexapeptide (or hexapeptide derivative), as it appears in the sequence from left to right.

Thus, an inhibitory peptide disclosed herein consists of, or comprises, a hexapeptide derivative having an amino acid sequence selected from the group consisting of SEQ ID Nos: 5-28, wherein the amino acid sequence comprises a single conservative amino acid substitution, or wherein the amino acid sequence comprises conservative amino acid substitutions at two of positions 1, 3 and 5 or at two of positions 2, 4 and 6.

In certain embodiments, an inhibitory peptide described herein consists of, or comprises, a hexapeptide derivative having an amino acid sequence selected from the group consisting of SEQ ID Nos: 6-7, 9-16, 18-19, and 21, 22-28, wherein the amino acid sequence comprises a single conservative amino acid substitution, or wherein the amino acid sequence comprises conservative amino acid substitutions at two of positions 1, 3 and 5 or at two of positions 2, 4 and 6. In particular, the inhibitory peptide may consist of, or comprise an amino acid sequence selected from the group consisting of SEQ ID Nos: 6-7, 9-16, wherein the amino acid sequence comprises a single conservative amino acid substitution, or wherein the amino acid sequence comprises conservative amino acid substitutions at two of positions 1, 3 and 5 or at two of positions 2, 4 and 6.

In certain embodiments, an inhibitory peptide described herein consists of, or comprises, a hexapeptide derivative having an amino acid sequence selected from the group consisting of SEQ ID Nos: 6-7, 9-15, 18-19, 21, and 24-28, wherein the amino acid sequence comprises a single conservative amino acid substitution, or wherein the amino acid sequence comprises conservative amino acid substitutions at two of positions 1, 3 and 5 or at two of positions 2, 4 and 6.

In certain embodiments, an inhibitory peptide described herein consists of, or comprises, a hexapeptide derivative having an amino acid sequence selected from the group consisting of SEQ ID Nos: 6, 7, 14-16, 18, 19, and 26-28, wherein the amino acid sequence comprises a single conservative amino acid substitution, or wherein the amino acid sequence comprises conservative amino acid substitutions at two of positions 1, 3 and 5 or at two of positions 2, 4 and 6. In particular, the inhibitory peptide may consist of, or comprise a hexapeptide derivative having an amino acid sequence selected from the group consisting of SEQ ID Nos: 6, 7, 14, 15, and 28, wherein the amino acid sequence comprises a single conservative amino acid substitution, or wherein the amino acid sequence comprises conservative amino acid substitutions at two of positions 1, 3 and 5 or at two of positions 2, 4 and 6. In certain embodiment, the inhibitory peptide consists of, or comprises, a hexapeptide derivative having amino acid sequence SEQ ID NO: 14 or SEQ ID NO: 16, wherein the amino acid sequence comprises a single conservative amino acid substitution, or wherein the amino acid sequence comprises conservative amino acid substitutions at two of positions 1, 3 and 5 or at two of positions 2, 4 and 6.

In certain embodiments, the hexapeptide derivative is modified to include modifications of termini such as acetylation and amidation, as described above.

In certain embodiments, the hexapeptide derivative is in a pharmaceutically acceptable salt, as described above.

## 3. Hexapeptide-Based Molecules

In certain embodiments, an inhibitory peptide which is used in a method of diagnosis and/or treatment of tauopathies according to the present invention consists of, or comprises, a hexapeptide-based molecule. As used herein, the term *"hexapeptide-based molecule"*, refers to a hexapeptide, or a hexapeptide derivative, as defined herein, which has been modified, for example chemically modified, while retaining its inhibitory activity towards the interactions of the PHF6 peptide within abnormal Tau protein. The term "hexapeptide-based molecule" also refers to a hexapeptide, or a hexapeptide derivative, as defined herein, which has been modified, for example chemically modified, while retaining its inhibitory activity towards the self-association process of the PHF6 peptide *in vitro*. In certain embodiments, the modification is such that the hexapeptide, or hexapeptide derivative, as defined herein, is linked to a heterologous moiety.

As used herein, the term *"linkage"* and associated terms such as "link" and "linked" refer to a connection between two groups or molecules (*e.g.*, between the hexapeptide and heterologous moiety). The connection can be accomplished covalently (by covalent chemical bonds) or non-covalently, for example by physical forces such as electrostatic bonds, hydrogen bonds, ionic bonds, van der Waals bonds, or hydrophobic/hydrophilic interactions. The connection may be direct or indirect. When indirect, the connection may be *via* a linker or spacer. The terms *"linker"* and *"spacer"* are used herein interchangeably, and refer to a molecule that joins two other molecules (*e.g.,* the hexapeptide and heterologous moiety) either covalently or non-covalently. Linkers and spacers, in particular peptidic linkers and spacers, used in peptide modifications are known in the art. The term *"heterologous moiety"*, as used herein, is synonymous with the term *"conjugate moiety"* and refers to any molecule (chemical or biochemical, naturally-occurring or non-coded) which is different from the hexapeptides described herein. The heterologous moiety to be linked to a hexapeptide, or hexapeptide derivative, disclosed herein is preferably chosen for its ability to optimize the hexapeptide's, or hexapeptide derivative's, potency, pharmacokinetic behavior, stability and/or other biological, physical, and chemical properties, or to confer new physicochemical or functional properties to the hexapeptide, or hexapeptide derivative. Thus, for example, the heterologous moiety may be selected for its ability (1) to increase the hexapeptide's, or hexapeptide derivative's, stability, for example by reducing susceptibility to proteolysis, (2) to increase cell penetration, (3) to confer detectability properties, (4) to direct the hexapeptide, or hexapeptide derivative, to a given cellular target or environment, and the like. Examples of heterologous moieties suitable to be linked to a hexapeptide, or hexapeptide derivative, described herein include, but are not limited to, detectable moieties, cell penetrating agents, stability enhancing moieties, blood-brain barrier shuttle moieties, tag allowing cellular degradation, and any other molecule or moiety capable of conferring a desirable property to the hexapeptide, or hexapeptide derivative.

*a. Detectable Moieties*. The heterologous moiety can be a detectable moiety or detectable label. Thus, in certain embodiments, an inhibitory peptide for use in a method of diagnosis and/or treatment of tauopathies according to the present invention, consists of, or comprises, a hexapeptide, or hexapeptide derivative, described herein that is labeled with a detectable agent or moiety. A detectable moiety may be selected among radioactive isotopes, antigenic determinants, enzymes, nucleic acids available for hybridization, chromophores, fluorophores, chemiluminescent molecules, electrochemically detectable molecules, and molecules that provide for altered fluorescence-polarization or altered light-scattering.

Preferably, the detectable moiety is suitable for detection by a diagnostic imaging technique, for example, fluorescence, positron emission tomography (PET), magnetic resonance imaging (MRI), single photon emission computed tomography (SPECT/CT), intravital laser scanning microscopy, endoscopy, and radiographic imaging. Useful detectable labels, depending on the technique or combination of imaging techniques used, include, but are not limited to, radionuclides, radiological contrast agents, paramagnetic ions, metals, biological tags, fluorescent labels, near infrared labels, chemiluminescent labels, ultrasound contrast agents, and photoactive agents. Such diagnostic agents are well known in the art and any such known diagnostic agent may be used. Non-limiting examples of diagnostic agents may include a radionuclide such as $^{110}$In, $^{111}$In , $^{177}$Lu, $^{18}$F, $^{52}$Fe, $^{62}$Cu, $^{64}$Cu, $^{67}$Cu, $^{67}$Ga, $^{68}$Ga, $^{44}$Sc, $^{43}$Sc, $^{47}$Sc, $^{86}$Y, $^{90}$Y, $^{89}$Zr, $^{94m}$Tc, $^{94}$Tc, $^{99m}$Tc, $^{120}$I, $^{123}$I, $^{124}$I, $^{125}$I, $^{131}$I, $^{154-158}$Gd, $^{32}$P, $^{11}$C, $^{13}$N, $^{15}$O, $^{186}$Re, $^{188}$Re, $^{51}$Mn, $^{52m}$Mn, $^{55}$Co, $^{72}$As, $^{75}$Br, $^{76}$Br, $^{82m}$Rb, $^{83}$Sr, or other gamma-, beta-, or positron-emitters. Suitable paramagnetic ions may include chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III) or erbium (III). Metal contrast agents may include lanthanum (III), gold (III), lead (II) or bismuth (III). Ultrasound contrast agents may comprise liposomes, such as gas filled liposomes. Radiopaque diagnostic agents may be selected from barium compounds, gallium compounds, and thallium compounds. A wide variety of fluorescent labels are known in the art, including but not limited to, fluorescein isothiocyanate, rhodamine, phycoerythrin, phycocyanin, allophycocyanin, o-phthaldehyde and fluorescamine. Chemiluminescent labels of use may include luminol, isoluminol, an aromatic acridinium ester, an imidazole, an acridinium salt or an oxalate ester.

In certain preferred embodiments, a hexapeptide, or hexapeptide derivative, described herein is labeled with a detectable moiety suitable for detection by positron emission tomography (PET), which is one of the main brain imaging techniques. In such embodiments, an inhibitory peptide consists of, or comprises, a hexapeptide, or hexapeptide derivative, described herein labeled with a radionuclide selected from the group consisting of $^{11}$C, $^{13}$N, $^{15}$O, $^{18}$F, $^{62}$Cu, $^{64}$Cu, $^{67}$Cu, $^{67}$Ga, $^{68}$Ga, $^{44}$Sc, $^{43}$Sc, $^{47}$Sc, $^{124}$I, $^{76}$Br, and $^{82}$Rb, in particular with any one of $^{62}$Cu, $^{64}$Cu, $^{67}$Cu, $^{67}$Ga, $^{68}$Ga, $^{44}$Sc, $^{43}$Sc, and $^{47}$Sc.

In certain embodiments, a chelating agent may be attached to a hexapeptide, or hexapeptide derivative, and used to chelate a detectable moiety, such as a radionuclide. Exemplary chelators include, but are not limited to, DTPA (such as Mx-DTPA),, DO3A, DOTA, EDTA, TETA, EHPG, HBED, NETA, NOTA, DOTMA, DFO, TETMA, PDTA, TTHA, LICAM, HYNIC, and MECAM. Methods of conjugation and use of chelating agents to attach metal or other ligands to proteins and peptides are well known in the art.

In certain embodiments, a hexapeptide, or hexapeptide derivative, described herein is attached to a nanoparticle suitable for use in medical imaging (Kalra et al., "10. Nanoparticles in Medical Imaging" in Nanoparticles in Analytical and Medical Devices, 2021, pages 175-210). As used herein, the term *"nanoparticle"* is meant to include particles, spheres, capsules, and other structures having a mean diameter of less than 1 micrometer. Preferred particles have a particle size up to about 50 nm, or up to about 10 nm.

Suitable nanoparticles include quantum dots, *i.e.,* bright, fluorescent nanocrystals with physical dimensions small enough such that the effect of quantum confinement gives rise to unique optical and electronic properties. Other suitable nanoparticles are optically detectable nanoparticles, such as metal nanoparticles. Metals used to form the nanoparticles include, but not limited to, Ag, Au, Cu, Al, Fe, Co, Ni, Ru, Rh, Pd, and Pt or oxides thereof. In particular, the metal may comprise Fe or iron oxide. A further surface functional layer can be added or formed in combination with a metal core material. Such functional layers can include, but are not limited to, Ag oxide, Au oxide, $SiO_2$, $Al_2O_3$, Si3N4, $TiO_2$, ZnO, $ZrO_2$, $HfO_2$, $Y_2O_3$, tin oxide, antimony oxide, iron oxide, and other oxides; Ag doped with chlorine or chloride, Au doped chlorine or chloride, Ethylene and Chlorotrifluoroethylene (ECTFE), Poly(ethylene-co-butyl acrylate-co-carbon monoxide) (PEBA), Poly(allylamine hydrochloride) (PAH), Polystyrene sulfonate (PSS), Polytetrafluoroethylene (PTFE), Polyvinyl alcohol (PVA), Polyvinyl chloride (PVC), Polyvinyldene fluoride (PVDF), Polyvinylpyrrolidone (PVP), and other polymers; stacked multiple layers at least two layers including above listed metal layers and non-metal layers, and the like. In some embodiments, the metal core can be Au, Ag, Fe, Ti, Ni, Cr, Pt, Ru, NiCr alloy, NiCrN, PtRh alloy, CuAuCo alloy, IrRh alloy and/or WRe alloy. The metal(s) used should be biocompatible. Other suitable nanoparticles include magnetic nanoparticles. The term *"magnetic nanoparticles"* refers to a magnetically responsive nanoparticles that contain one or more metals or oxides or hydroxides thereof. For example, superparamagnetic iron oxides (SPIOs) are a class of MR contrast agents composed of nanoparticles of iron oxide crystals coated in carbohydrates. As known in the art, optically detectable metal nanoparticles or quantum dots can be detected *in vivo* upon systemic administration to a subject using magnetic resonance imaging (MRI), magnetic resonance spectroscopy (MRS), nuclear magnetic resonance imaging (NMR), multimodal imaging, fluorescent, positron emission tomography (PET), near infrared (NIR) imaging, X-ray imaging, and computed tomography (CT).

In certain embodiments, the nanoparticle is a multimodal nanoparticle, in particular a multimodal nanoparticle suitable for imaging of brain tissues. The term *"multimodal nanoparticle",* as used herein, refers to a nanoparticle that can be used in combination with more than one bioimaging technique. A multimodal nanoparticle can have multiple moieties which behave as different contrast agents for different imaging techniques. Multimodal imaging or multiplexed imaging refers to simultaneous production of signals from more than one imaging technique. For example, one could develop multimodal nanoparticles using a combination of optical, magnetic, and/or radioactive reporters to be detected by SPECT, MRI, and PET. Multimodal nanoparticles are known in the art (Jarzyna et al., Rev. Nanomed. Nanobiotechnol., 2010, 2(2): 138-150; Huang et al., Dalton Trans., 2011, 40(23): 6087-6103; Madru et al., J. Nucl. Med., 2012, 53(3): 459; Tang et al., Nanomedicine, 2015, 10(8): 1343-1359; Burke et al., Philos. Trans. A Math. Phys. Eng. Sci., 2017, 375(2107): 20170261; Tang, "PET/SPECT/MRI multimodal nanoparticles, in "Design and Applications of Nanoparticles in Biomedical Imaging", Bulte and Modo (Eds), 2017, pages 205-228). In particular, nanoparticle-based radiotracers show promise as multimodality probes for SPECT/MRI and PET/MRI.

*b. Cell Penetrating Agent.* The heterologous moiety can be a cell penetrating agent, such as a cell penetrating peptide (*i.e.,* a CPP). Thus, in certain embodiments, an inhibitory peptide according to the present invention consists of, or comprises, a hexapeptide, or hexapeptide derivative, described herein that is directly or indirectly linked to a cell penetrating agent, for example a cell penetrating peptide.

As used herein, the term *"cell penetrating peptide"* refers to a short peptidic agent (generally of less than 30 amino acids) which enhances translocation of any of the attached hexapeptides across a cell membrane. Typically, cell penetrating peptides belong to three different classes: the polycationic CPPs, which have an amino acid composition containing a high relative abundance of positively charged amino acid residues such as lysine or arginine; the amphipathic CPPs, which have sequences that contain an alternating pattern of polar/charged amino acid residues and nonpolar/hydrophobic amino acid residues; and the hydrophobic CPPs, which have sequences containing only apolar residues, with low net charge or have hydrophobic amino acid groups that are crucial for cellular uptake.

Examples of typical CPPs suitable for use in the practice of the present invention include, but are not limited to, polyarginines (PolyARG) (nR, wherein n is an integer such as 4<n<17, in particular 9-Arginine, 8-Arginine, 6-Arginine), polyLYS (nK, wherein n is an integer such as 4<n<17), D-polyARG (nR, wherein n is an integer such as 4<n<17), D-polyLYS (nK, wherein n is an integer such as 4<n<17), SynB1, SynB3, Penetratin, Drosophila homeotic protein antennapedida (ANTp), PenArg, PenLys, TatP59W, Tat (trans-activator of transcription of HIV), Tat (48-60), R9-Tat, D-Tat, BMVGag (7-25), FHVCoat(35-49), HTLV-II Rex(4-16), P22 N-Q(4-30), pVEC, TP10, PTD-4, PTD-5, Pep-1, Pep-2, Pep-3, E NQ-22), B21 N-(12-29), U2AFQ(42-153), PRP6(129-144), MAP, SBP, FBP, MPG, MPG(ANLS), and REV(34-50), which are described in WO 2018/005867; and EB1, Transportan, p-Antp, hcT(18-32), Xentry and KLA seq. Other examples of cell-penetrating peptides include W/R, NLS (nuclear localization signal), $AlkCWK_{18}$, $DiCWK_{18}$, DipaLytic, $K_{16}RGD$, P1, P2, P3, P3a, P9.3, Plae, Kplae, cKplae, MGP, HA2, $LARL_{46}$, (LARL)n, Hel-11-7, KK, KWK, RWR, Herpes virus VP22, SCWKn, RGD, MPG, ARF (1-22), BPrPp (1-28), VT5, MAP, SG3, Pep-7, stapled peptides, prenylated peptides, pepducins, R6W3, arginine-rich peptides like $(Arg-X-Arg)_n$ peptides (where X is a generic carbon chain spacer), proline-rich peptides, (Schwartz et al., Curr. Opin. Mol. Ther., 2000, 2(2): 162-7; Lee et al., Methods Mol. Biol., 2013, 991: 281-92; Bechara et al., FEBS Lett., 2013, 587(12): 1693-1702; Di Pisa et al., J. Pept. Sci., 2015, 21(5):356-369; Guo et al., Biomed. Rep., 2016, 4(5): 528-534). According to a particular aspect, a cell penetrating moiety is as described in Svensen et al., 2012, Trends in Pharmacological Sciences, 33(4): 186-192.

In certain embodiments, the cell-penetrating peptidic moiety linked to a hexapeptide, or hexapeptide derivative, described herein is a polyLys sequence, which allows, after binding, the targeting of free Tau proteins in the cytoplasm to cell degradation systems (such as proteasome or other...).

In certain embodiments, the CPP is directly coupled to the hexapeptide, or hexapeptide derivative. In other embodiments, it is desirable to separate a highly charged CPP from the hexapeptide, or hexapeptide derivative, with a linker to allow the hexapeptide to retain its inhibitory activity. Any of a variety of linkers can be used. Suitable linkers include peptidic sequence consisting of 1 to 10 (e.g., 1, 2, 3, 4, 5, 6, 7, 8, 9, 10) amino acid residues.

Another method of enhancing cell penetration of a peptide is via N-terminal myristoilation. Thus, in certain embodiments, an inhibitory peptide consists of, or comprises, a hexapeptide, or hexapeptide derivative, described herein that is linked to a myristoyl group (derived from myristic acid). The myristoyl group is a 14-carbon saturated fatty acid (C14), which gives the peptide sufficient hydrophobicity and affinity for membranes, but not enough to permanently anchor the peptide in the membrane. Generally, myristoylation therefore acts as a conformational localization switch, in which protein conformational changes influence affinity of a peptide for membrane attachment. The myristoyl group may be covalently attached via an amide bond to the alpha-amino group of the N-terminal amino acid of the hexapeptide.

*c. Stability Enhancing Moieties*. The heterologous moiety can be a stability enhancing moiety. Thus, in certain embodiments, an inhibitory peptide for use in a method according to the present invention consists of, or comprises, a hexapeptide, or hexapeptide derivative, described herein that is directly or indirectly linked to a stability enhancing moiety.

As used herein, the term *"stability enhancing moiety"* refers to a moiety (molecule) which, when linked to a hexapeptide, or hexapeptide derivative, described herein, affects the pharmacokinetics thereof; more specifically it increases the hexapeptide's, or hexapeptide derivative's, metabolic stability and plasma half-life. Stability enhancing moiety may also influence the hexapeptide's, or hexapeptide derivative's, solubility, gut permeability, resistance to proteases and the like. Stability enhancing moieties include, but are not limited to, carbohydrates (glycans), lipids, such as fatty acids, and water-soluble polymers, such as polyethylene glycol (PEG).

The introduction of carbohydrate moieties (glycation) is known to change the physiological properties of peptides, as they can improve their bioavailability. The favorable impact of glycosylation on pharmacokinetics properties of the peptide leads to an increase in their oral absorption and bioavailability. In addition to O- and N-linked glycosylation approaches, several chemical methods and chemo-enzymatic approaches have been established for the attachment of carbohydrate units to different amino acid residues at the N-terminus of the peptide's sequence. The carbohydrate may be a monosaccharide (e.g., glucose, galactose, fructose), a disaccharide (e.g., sucrose, lactose, maltose), an oligosaccharide (e.g., raffinose, stachyose), or a polysaccharide (a starch, amylase, amylopectin, cellulose, chitin, callose, laminarin, xylan, mannan, fucoidan, or galactomannan).

Lipid conjugates of peptides are known to exhibit higher stability and longer plasma half-lives. Examples of suitable lipids include, but are not limited to, a fatty acid, an eicosanoid (e.g., prostaglandin, leukotriene, thromboxane), a glycerolipid (e.g., mono-, di-, tri-substituted glycerols), a glycerophospholipid (e.g., phosphatidylcholine, phosphatidylinositol, phosphatidylethanolamine, phosphatidylserine), a sphingolipid (e.g., sphingosine, ceramide), a sterol lipid (e.g., steroid, cholesterol), a prenol lipid, a saccharolipid, or a polyketide, oil, wax, cholesterol, sterol, fat-soluble vitamin, monoglyceride, diglyceride, triglyceride, or a phospholipid. In certain embodiments, the lipid is a fatty acid. A fatty acid is a carboxylic acid with a long aliphatic chain, which is either saturated or unsaturated. Preferred fatty acids have an unbranched chain of an even number of carbon atoms, from 4 to 28. For fatty acid conjugation various approaches are available. Synthesis can be performed where fatty acids are either conjugated to the N-terminus, or to the side-chain of a lysine. Also, the cysteine residues in peptides, or in a linker or spacer, can be modified by fatty acids, giving the corresponding thioester derivatives. The fatty acids that are most commonly used for lipidation (fatty acids conjugation to peptides) are: caprylic acid (C8), capric acid (C10), lauric acid (C12), myristic acid (C14), palmitic acid (C16) and stearic acid (C18).

Covalent attachment of a peptide to a water soluble (or hydrophilic) polymer is known to affect peptide pharmacokinetics, more specifically it increases peptide stability and plasma half-life. Examples of suitable hydrophilic polymers include, but are not limited to, polyethylene glycols, polyvinyl alcohol, other poly(alkylene oxides) such as poly(propylene glycol) and the like, poly(oxyethylated polyols) such as poly(oxyethylated glycerol) and the like, carboxymethylcellulose, dextran, polyvinyl alcohol, polyvinyl pyrrolidone, poly-1,3-dioxolane, poly-1,3,6-trioxane, ethylene/maleic anhydride, and polyaminoacids. Linear or branched hydrophilic polymers are contemplated.

In certain embodiments, PEG, which is a well-known polymer with good solubility in many aqueous and organic solvents and which exhibits low toxicity and lack of immunogenicity, and is clear, colorless, odorless, and stable, is preferably used as hydrophilic polymer. PEG's most common form is a linear or branched polyether with terminal hydroxyl groups: $HO-(CH_2CH_2O)_n-CH_2CH_2-OH$. Monofunctional methoxy-PEG (mPEG) may be preferred for peptide modification: $CH_3O-(CH_2CH_2O)_n-CH2CH2-OH$, as it can be derivatized with a number of linkage moieties, yielding methoxyPEG-amines, -maleimides, or -carboxylic acids. Preferably, PEGs with an average molecular weight of from about 5 to about 50 kDa are used in clinical and approved pharmaceutical applications. Two or more lower-weight chains can be added to increase the total molecular weight of the PEG complex. PEGylation of peptides is widely known in the state of the art (for a review see, Veronese, Biomaterials, 2001, 22: 405-417). PEG can be coupled to different available reactive groups on the peptide or a linker, such as lysine, aspartic acid, cysteine, glutamic acid, serine, threonine, the N-terminal amine and the C-terminal carboxylic acid or other specific sites.

*d. Blood-Brain Barrier Shuttle Moieties.* The heterologous moiety can be a blood-brain barrier shuttle moiety. Thus, in certain embodiments, an inhibitory peptide for use in a method according to the present invention consists of, or comprises, a hexapeptide, or hexapeptide derivative, described herein that is directly or indirectly linked to a blood-brain barrier shuttle moiety.

As used herein, the term *"blood-brain barrier shuttle moiety"* refers to a moiety which, when linked to a hexapeptide, or hexapeptide derivative, described herein, has the ability to carry the peptide across the blood-brain barrier (BBB) either through an active or passive transport mechanism. A BBB shuttle is a powerful facilitator of drug delivery to the brain. In certain embodiments, the blood-brain barrier shuttle moiety is a BBB shuttle peptide. Examples of suitable BBB shuttle peptides include, but are not limited to, Angiopep-2 (Demeule et al., J. Pharmacol. Exp. Ther., 2008, 324: 1064-1072; Demeule et al., J. Neurochem., 2008, 106: 1534-1544; Ying et al., Angew. Chem., Int. Ed., 2014, 53: 12436-12440); ApoB (3371-3409) (Spencer et al., Proc. Natl. Acad. Sci. U.S.A., 2007, 104: 7594-7599; Sorrentino et al., EMBO Mol. Med., 2013, 5: 675-690); ApoE (159-167) (Wang et al., Proc. Natl. Acad. Sci. U. S. A., 2013, 110: 2999-3004; Böckenhoff et al., J. Neurosci., 2014, 34: 3122-3129; Re et al., Nanomedicine, 2011, 7: 551-559); Peptide-22 (Malcor et al., J. Med. Chem., 2012, 55: 2227-2241); THR (Lee et al., Eur. J. Biochem., 2001, 268: 2004-2012; Prades et al., Biomaterials, 2012, 33: 7194-7205); THR retro-enantio (Prades et al., Angew. Chem., Int. Ed., 2015, 54: 3967-3972); CRT (Staquicini et al., J. Clin. Invest., 2011, 121: 161-173); Leptin30 (Barrett et al., Regul. Pept., 2009, 155: 55-61; Liu et al., Biomaterials, 2010, 31: 5246-5257); RVG29 (Kumar et α/., Nature, 2007, 448: 39-43; Kim et al., Mol. Ther., 2010, 18: 993-1001; Zadran et al., NeuroMol. Med., 2013, 15: 74-81); [D]CDX (Wei et al., Angew. Chem., Int. Ed., 2015, 54: 3023-3027); Apamin (Oller-Salvia et al., Biopolymers, 2013, 100: 675-686; Wu et al., Mol. Pharmaceutics, 2014, 11: 3210-3222); MiniAp-4 (Oller-Salvia et al., Angew. Chem., Int. Ed., 2016, 55: 572-575); GSH (Gaillard et al., J. Controlled Release, 2012, 164: 364-369; Mdzinarishvili et al., Drug Delivery Transl. Res., 2013, 3: 309-317; Lee et al., J. Neuroimmunol., 2014, 274: 96-101; Lindqvist et al., Mol. Pharmaceutics, 2013, 10: 1533-1541); G23 (Georgieva et al., Angew. Chem., Int. Ed., 2012, 51: 8339-8342); g7 (Costantino et al., J. Controlled Release, 2005, 108: 84-96; Tosi et al., Curr. Med. Chem., 2013, 20: 2212-2225; Tosi et al., J. Neural Transm., 2011, 118: 145-153; Vilella et al., J. Controlled Release, 2014, 174: 195-201); TGN (Li et al., Biomaterials, 2011, 32: 4943-4950; Gao et al., Biomaterials, 2012, 33: 5115-5123; Zhang et al., Biomaterials, 2014, 35: 456-465); TAT (47-57) (Schwarze et al., Science, 1999, 285: 156915-72; Aarts et al., Science, 2002, 298: 846-850; Wang et al., Biomaterials, 2010, 31: 2874-2881); SynB1 (Rousselle et al., Mol. Pharmacol., 2000, 57: 679-686; Drin et al., J. Biol. Chem., 2003, 278: 31192-3120); Diketopiperazines (Teixidó et al., J. Am. Chem. Soc., 2007, 129: 11802-11813; Teixidó et al., Biopolymers, 2013, 100: 662-674); and PhPro (Arranz-Gibert et al., J. Am. Chem. Soc., 2015, 137: 7357-7364), which are all described in Oller-Salvia et al., Chem. Soc. Rev., 2016, 45: 4690-4707). Other examples of BBB shuttle peptides include, but are not limited to, the BBN shuttle peptides described in Diaz-Perlas et al., Chem. Sci., 2018, 9: 8409-8415 and Majerova et al., Molecules, 2020, 25(4): 874.

*e. Other Heterologous Moieties.* Other examples of heterologous moieties suitable to be attached to a hexapeptide, or hexapeptide derivative, described herein include albumin-binding small molecules, which can reduce glomerular filtration, improve proteolytic stability, and prolong half-life by indirectly interacting with albumin through the highly bound small molecules. Other suitable heterologous moieties include, but are not limited to, albumin (in particular human serum albumin or HAS) or IgG fragments (*e.g.*, variable region, CDR, or Fc region) that are known to reduce clearance and prolong half-life of peptides. Fusion of a peptide with hydrophilic amino acid polymers is also known to extend the peptide's half-life, Hydrophilic amino acid polymers include, but are not limited to, XTEN polymers (non-immunogenic polypeptides developed by AMunix and consisting of the six hydrophilic, chemically stables amino acids residues A, E, G, P, S, and T) or PAS polymers (polypeptides, developed by XL-Protein GmbH, which comprise long repetitive sequences of the small L-amino acids P, A and/or S).

*f. Peptide Modifications.* Alternatively, or in addition to being linked to a heterologous moiety, a hexapeptide, or hexapeptide derivative, as described herein may itself be modified to optimize the hexapeptide's potency, pharmacokinetic behavior, stability and/or other biological, physical and chemical properties.

For example, a hexapeptide, or hexapeptide derivative, disclosed herein may be modified to increase resistance to proteolytic breakage when present in a proteolytic enriched environment, e.g., in the blood. Indeed, a number of proteolytic enzymes in blood/plasma, liver or kidney are exopeptidases, aminopeptidases and carboxypeptidases and they break down peptide sequences from the N- and C-termini. Modification of the N- and/or C-termini can often improve peptide stability. It has often been reported that N-acetylation and C-amidation increase resistance to proteolysis. Other N-terminal modifications include methylation, acylation and thioglycolic acid amidation.

In certain embodiments, a hexapeptide, or hexapeptide derivative, described herein is modified to contain, at the N-terminal portion, one or two additional amino acid residues that achieve resistance to peptidase cleavage. The additional amino acid residue at position 1 may be selected from the group consisting of: D-histidine, desaminohistidine, hydroxyl-histidine, acetyl-histidine, homo-histidine, N-methyl histidine, $\alpha$-methyl histidine, imidazole acetic acid, or $\alpha,\alpha$-dimethyl imidazole acetic acid (DMIA). The additional amino acid residue at position 2 may be selected from the group consisting of: D-serine, D-alanine, valine, glycine, N-methyl serine, N-methyl alanine, or $\alpha$-aminoisobutyric acid.

In certain embodiments, a hexapeptide, or hexapeptide derivative, described herein may be modified by additions that introduce a charged amino acid into the C-terminal portion. Such modifications enhance stability and solubility. The terms *"charged amino acid"* and *"charged residue"* are used herein interchangeably, and refer to an amino acid that comprises a side chain that is negatively-charged (*i.e.,* deprotonated) or positively-charged (*i.e.,* protonated) in aqueous solution a physiological pH. One, two or three (and in some instances, more than three) charged amino acids, in particular negatively-charged amino acids, may be introduced at the C-terminal position. Negatively-charged amino acid residues include aspartic acid, glutamic acid, cysteic acid, homocysteic acid and homoglutaminic acid. Such modifications increase solubility.

A hexapeptide, or hexapeptide derivative, described herein can alternatively be stabilized through cyclization, where cyclization does not severely interfere with peptide characteristics, in particular with the hexapeptide's ability to inhibit, reduce or prevent the interactions of the PHF6 peptide. Cyclic peptides are polypeptide chains whose amino and carboxyl termini are linked together with a peptide bond or other covalent bond, forming a circular chain. Cyclization introduces conformational constraint, reduces flexibility of peptides and thereby can improve stability and permeability. Depending on the functional groups, peptides can be cyclized head-to-tail, head/tail-to-side-chain, or side-chain-to-side-chain. Cyclization can take place *via* the formation of any one of amide, thioether, thioester, urea, carbamate, sulfonamide, lactam, lactone, disulfide bridges, and the like. For example, a hexapeptide, or hexapeptide derivative, described herein may be modified to contain amino and carboxyl terminal cysteine amino acid residues, which facilitate disulfide bond formation. Thus, a hexapeptide, or hexapeptide derivative, may be modified to contain additional cysteine amino acid residues, wherein the cysteine amino acid residues are near the termini but not necessarily at the very end. Alternatively, the cysteine amino acid residues may be located within five amino acid residues at the termini of the hexapeptide, or hexapeptide derivative. Disulfide bridges are known to create folding and conformational constraints that can improve potency, selectivity, and stability of peptides. In a different method, a peptide may be cyclized *via* a bifunctional agent, such as a dicarboxylic acid (*e.g.*, octanedioic acid), which introduces a link between two functional groups (*e.g.,* free amino, hydroxyl, thio, and the like) of the hexapeptide, or hexapeptide derivative. Methods of design and synthesis of cyclic peptides are well-known in the art.

*g. Inhibitory Peptides.* As will be recognized by one skilled in the art, an inhibitory peptide for use in a method according to the present invention consists of a hexapeptide, or hexapeptide derivative, described herein that has been modified to contain one, or more than one, of the different heterologous moieties and hexapeptide modifications disclosed herein as long as such modifications do not significantly affect the inhibitory peptide's functionality in performing its desired role and providing its intended diagnostic and/or therapeutic effects. In other words, within a given hexapeptide-based molecule, a modification or a combination of modifications is only acceptable to the extent that the resulting peptide retains the inhibitory property of the starting hexapeptide sequence towards the self-association process of abnormal Tau protein, in particular towards the assembly of abnormal Tau into paired helical filaments (PHFs), which depends on PHF6. A modification or a combination of modifications of a hexapeptide, or hexapeptide derivative, described herein may be experimentally determined to be acceptable if the resulting peptide retains the inhibitory property of the starting hexapeptide sequence, or hexapeptide derivative sequence, towards the self-association process of the PHF6 peptide *in vitro.* In some embodiments, about 100% of the activity is retained in a given assay. In other embodiments, about 98%, about 95%, about 90%, about 85%, about 80%, about 70%, or about 60% of the activity is retained in the assay.

Depending on the intended use of the resulting inhibitory peptide, one skilled in the art may easily conceive an optimized combination of hexapeptide modifications (*i.e.,* attachment to a heterologous moiety and/or hexapeptide modification). For example, when an inhibitory peptide is intended to be used in a method of diagnostic of tauopathies, the hexapeptide-based molecule may preferably comprise a hexapeptide labeled with a detectable moiety, preferably with a radionuclide, in particular a radionuclide suitable for detection by PET or a multimodal imaging technique.

Thus, in certain embodiments, an inhibitory peptide for use in a method of diagnosis and treatment of tauopathies consists of a hexapeptide, or hexapeptide derivative, described herein labeled, at the C-terminus, with a detectable moiety, for example a radionuclide suitable for detection by PET and modified to be linked, at the N-terminus, to a stabilizing moiety or a CPP moiety or a BBB shuttle moiety.

Inhibitory peptides described herein specifically interact with the PHF6 sequence. In particular, the inhibitory peptides described herein specifically (*i.e.,* selectively, preferentially) bind to the PHF6 sequence rather than to unintended sequences. The PHF6 sequence to which an inhibitory peptide binds may be the PHF6 peptide *in vitro,* or may be part of an abnormal Tau protein in cell culture or *in vivo,* wherein the abnormal Tau protein may be in the form of a monomer, a small aggregate, an oligomer, or a fibril. For example, the binding can be 2 times, 5 times, 10 times, 100 times, 200 times, 500 times or more than 500 times stronger, or no binding at all can be detected to an unintended target. Conventional methods can be used to determine the specificity of binding, such as e.g., competitive binding assays or other suitable analytic method.

## 3. Preparation of the L-Hexapeptides and Hexapeptide-Based Molecules

The hexapeptides, hexapeptide derivatives, and hexapeptide-based molecules disclosed herein may be prepared using any of a variety of art-recognized methods, including chemical synthesis and recombinant expression in a suitable host cell.

For example, the hexapeptides and derivatives thereof may be prepared using standard chemical methods. Solid-phase peptide synthesis, which was initially described by R.B. Merrifield (J. Am. Chem. Soc. 1963, 85: 2149-2154), is a quick and easy approach to synthesizing peptides and peptidic molecules of known short sequences. A compilation of such solid-state techniques may be found, for example, in "Solid Phase Peptide Synthesis" (Methods in Enzymology, G.B. Fields (Ed.), 1997, Academic Press: San Diego, CA, which is incorporated herein by reference in its entirety). Most of these synthetic procedures involve the sequential addition of one or more amino acid residues or suitably protected amino acid residues to a growing peptide chain. Once the desired peptide is assembled, it is cleaved off from the solid support, precipitated, and the resulting free peptide may be analyzed and/or purified as desired. Solution methods, as described, for example, in "The Proteins" (Vol. II, 3rd Ed., H. Neurath et al. (Eds.), 1976, Academic Press: New York, NY, pp. 105-237), may also be used to synthesize the hexapeptides disclosed herein.

Alternatively, in order to generate sufficient quantities of hexapeptides, hexapeptide derivatives, or hexapeptide-based molecules for use in methods according to the present invention, one can use recombinant DNA methods (Sambrook et al., Molecular Cloning: A Laboratory Manual, 3rd ed., Cold Spring Harbor Press, Cold Spring Harbor, N.Y. 2001; and Ausubel et al., Current Protocols in Molecular Biology, Greene Publishing Associates and John Wiley & Sons, N.Y., 1994). These methods generally involve generation of a nucleic acid (*e.g.,* DNA) encoding the peptide of interest, transfer of the peptide-encoding nucleic acid molecule into a suitable vector, and bulk expression in a cell culture system. The DNA coding sequences for the hexapeptides, or hexapeptide derivatives, may be readily prepared synthetically using methods known in the art (see, for example, M.P. Edge et al., Nature, 1981, 292: 756-762). Suitable recombinant expression vectors include, without limitation, plasmid vectors, viral vectors, including phage vectors, artificial vectors, yeast vectors, eukaryotic vectors, etc., which contain the necessary genetic elements to direct expression of the coding sequence of interest. Insertion of the nucleic acid molecule into the expression vector results in the coding sequence being operatively linked to the necessary regulatory sequences. Expression systems containing the requisite control sequences, such as promoters and polyadenylation signals, and preferably enhancers, are readily available for a variety of hosts (see, for example, Sambrook et al., Molecular Cloning: A Laboratory Manual, 2nd Ed., 1989, Cold Spring Harbor Press: Cold Spring, NY; and R. Kaufman, Methods in Enzymology, 1990, 185: 537-566). The transformed host cells are then cultured and maintained under conditions favoring expression of the desired peptide. The peptide thus produced is recovered and isolated, either directly from the culture medium or by lysis of the cells.

Recombinant techniques may be preferred when the hexapeptide, or hexapeptide derivative, is linked to a heterologous peptidic moiety since recombinant techniques are better suited for generation of relatively long polypeptides (*e.g.,* longer than 10 or 20 amino acids) and large amounts thereof. Thus, as understood by one skilled in the art, a hexapeptide, or hexapeptide derivative, or hexapeptide-based molecule described herein may be produced as a fusion protein (*i.e.,* a molecule in which the hexapeptide sequence is linked to a fusion partner). The term ***"fusion partner"*** refers to an amino acid sequence that confers to the fusion protein one or more desirable properties. Thus, a fusion partner may be an amino acid sequence that improves the expression of the hexapeptide, or hexapeptide derivative, or hexapeptide-based molecule in host cells during preparation of the fusion protein, and/or an amino acid sequence that facilitates purification of the fusion protein, and/or an amino acid sequence that increases the stability of the fusion protein compared to the stability of the non-fused protein (*e.g.,* to obtain a fusion protein with increased stability to proteases), and the likes. Examples of suitable fusion partners include, for example, polyhistidine tags, that allow for the easy purification of the resulting fusion protein on a nickel chelating column. Glutathione-S-transferase (GST), maltose B binding protein, or protein A are other examples of suitable fusion partners that can be fused to a hexapeptide described herein using commercial fusion expression vectors. The fusion partner may also be heterologous peptide moiety as defined above.

Hexapeptides, derivatives thereof, and hexapeptide-based molecules according to the present invention can be purified using a variety of standard protein purification techniques, including, but not limited to, affinity chromatography, ion exchange chromatography, filtration, electrophoresis, hydrophobic interaction chromatography, gel filtration chromatography, reverse phase chromatography, concanavalin A chromatography, chromatofocusing, differential solubilization, and the like.

It is recognized that "purity" is a relative term, and not to be necessarily construed as absolute purity or absolute enrichment or absolute selection. In some embodiments, the purity of the retrieved hexapeptide, hexapeptide derivative, or hexapeptide-based molecule is at least or about 60%, at least or about 70%, at least or about 80%, or at least or about 90% (*e.g.,* at least or about 91%, at least or about 92%, at least or about 93%, at least or about 94%, at least or about 95%, at least or about 96%, at least or about 97%, at least or about 98%, at least or about 99%) or is approximately 100%. In some preferred embodiments, the hexapeptides, hexapeptide derivatives, hexapeptide-based molecules described herein are preferably retrieved in "substantially pure form". As used herein, the term "substantially pure" refers to a purity that allows for the effective use of the inhibitory peptide in the applications to which it is intended. In diagnostic and/or treatment applications in humans, substantially pure preferably refers to at least or about 97%, at least or about 98%, at least or about 99%, or approximately 100% pure.

If desired, after preparation, a hexapeptide, hexapeptide derivative, or hexapeptide-based molecule may be sterilized. Sterilization may be carried out using any of a wide variety of sterilization techniques known in the art, for example, by filtration through a bacteria-retaining filter, by gamma irradiation, by electron-beam irradiation, or by incorporating sterilizing agents in the form of a sterile solid compositions which can be dissolved or dispersed in sterile water or other sterile injectable medium prior to use. Alternatively, or additionally, the purified hexapeptide, hexapeptide derivative, or hexapeptide-based molecule may be lyophilized prior to use.

The hexapeptides, hexapeptide derivatives, and hexapeptide-based molecules can be commercially synthesized by specialized company, such as Proteogenix (Schiltigheim, France), Genepep (Montpellier, France), Genscript (Piscataway, N.J.), New England Peptide (Gardner, Mass.), and CPC Scientific (Sunnyvale, Calif.), Peptide Technologies Corp. (Gaithersburg, Md.), and Multiple Peptide Systems (San Diego, Calif.).

Hexapeptides, hexapeptide derivatives, and hexapeptide-based molecules may be stored under appropriate conditions, either in a liquid form or a solid form, as known in the art. For example, after preparation/purification or upon receipt from a provider, peptides are to be kept in a cool, dark place. For best preservation, they can be stored under refrigeration at 4°C or colder, away from bright light. Dry peptides are stable at room temperature for days to weeks, but for long term storage, -20°C is preferred.

## 4. Combinations of Inhibitory Peptides

An inhibitory peptide described herein may be used alone in a method according to the invention. Alternatively, at least two inhibitory peptides described herein may be used in combination. Thus, in certain embodiments, a combination according to the present invention consists of two inhibitory peptides described herein. In other embodiments, a combination according to the present invention consists of more than two, for example three or four, inhibitory peptides described herein.

In particular, a combination for use in a method according to the present invention consists of at least two different inhibitory peptides described herein, wherein each inhibitory peptide is selected from the group consisting of inhibitory peptides consisting of, or comprising, any one of hexapeptides **4p** (SEQ ID NO: 6), **4pinv** (SEQ ID NO: 18), **5p** (SEQ ID NO: 7), **5pinv** (SEQ ID NO: 19), **8p** (SEQ ID NO: 9), **8pinv** (SEQ ID NO: 21), **9p** (SEQ ID NO: 10), **9pinv** (SEQ ID NO: 22), **13p** (SEQ ID NO: 11), **13pinv** (SEQ ID NO: 23), **14p** (SEQ ID NO: 12), **14p$_{inv}$** (SEQ ID NO: 24), **23p** (SEQ ID NO: 13), **23pinv** (SEQ ID NO: 25), **1tp** (SEQ ID NO: 14), **1tp$_{inv}$** (SEQ ID NO: 26), **4tp** (SEQ ID NO: 15), **4tpinv** (SEQ ID NO: 27), **6tp** (SEQ ID NO: 16), and **6tpinv** (SEQ ID NO: 28), and hexapeptide derivatives thereof as described herein. In particular, the combination may consist of at least two different inhibitory peptides, wherein each inhibitory peptide is selected from the group consisting of inhibitory peptides consisting of, or comprising, any one of hexapeptides **4p, 5p, 8p, 9p, 13p, 14p, 23p, 1tp, 4tp,** and **6tp,** and hexapeptide derivatives thereof as described herein.

In certain preferred embodiments, a combination consists of at least two different inhibitory peptides, wherein each inhibitory peptide is selected from the group consisting of inhibitory peptides consisting of, or comprising, any one of hexapeptides **4p, 4pinv, 5p, 5pinv, 1tp**, **1tp$_{inv}$,** **4tp, 4tpinv, 6tp,** and **6tpinv,** and hexapeptide derivatives thereof as described herein. In particular, the combination may consist of at least two different inhibitory peptides, wherein each inhibitory peptide is selected from the group consisting of inhibitory peptides consisting of, or comprising, any one of hexapeptides **4p, 5p, 1tp**, **4tp,** and **6tp,** and hexapeptide derivatives thereof as described herein.

In certain embodiments, the relative amounts of a first and a second (and a third, etc.....) inhibitory peptides within the composition are selected to affect the ability of the composition to inhibit one or more discreet phenomena observed in pathological Tau aggregation (*e.g.*, inhibition of PHF6 self-association, etc....). For example, for a binary combination, the relative amounts may be about 35:65; or about 40:60, or about 45:55, about 50:50, or about 55:45, or about 60:40, or about 65:35.

## II - Uses of the Inhibitory Hexapeptides and Hexapeptide-Based Molecules

As already mentioned above, the hexapeptide sequences provided herein specifically interact with (*i.e.,* specifically binds to) the PHF6 peptide, which is essential to the process of nucleation and fibrillation of abnormal Tau protein in the form of paired helical filament (PHF) and straight filament (SF) found in Alzheimer's disease and in the form of narrow Pick filament (NPH) and wide Pick filament (WPF) in Pick's disease. Due to loss of function or deregulation, the abnormal Tau protein, which comprises the PHF6 sequence, withdraws from the microtubule and is found free in the cytoplasm, thereby becoming accessible to inhibitory peptides. Thus, the inhibitory peptides of the invention may be used in a variety of applications, including therapeutic and diagnostic applications in the field of tauopathies and of disorders associated with abnormal Tau aggregation.

### 1 - Inhibitory / Therapeutic Applications

The present invention relates to the use of an inhibitory peptide described herein in a method for inhibiting the self-associative process of the PHF6 peptide *in vitro,* the method comprising steps of: contacting the PHF6 peptide, or a sample comprising the PHF6 peptide, with an effective amount of an inhibitory peptide under conditions allowing the inhibitory peptide to bind to the PHF6 peptide so that the self-associative process of the PHF6 peptide is inhibited.

The inhibitory peptide used in the method may be one of the inhibitory peptides described herein or a combination of at least two of the inhibitory peptides described herein. The method is carried out *in vitro* in solution, *e.g.,* using a model developed by the present Inventors (see Examples section). Contacting the PHF6 peptide, or a sample thereof, with an effective amount of the inhibitory peptide typically includes combining, mixing, or incubating the PHF6 peptide, or sample thereof, with the inhibitory peptide. It is within the abilities of one skilled in the art to determine the optimal conditions (time, temperature, pH, etc...) allowing the inhibitory peptide to bind to the PHF6 peptide so that the self-associative process of the PHF6 peptide is inhibited.

It will be recognized by one skilled in the art that such a method may be used to for inhibiting an associative process of the PHF6 peptide *in vitro,* wherein the associative process is the interaction of the PHF6 peptide with [374]HKLTF[378] (SEQ ID NO: 3), or the interaction of the PHF6 peptide with [336]QVEVKS[341] (SEQ ID NO: 4). In such cases, the *in vitro* model to be used is the VQIVYK/HKLTF interaction model and the VQIVYK/ QVEVKS interaction model, respectively, that the present Inventors are in the process of developing.

The present invention further relates to the use of an inhibitory peptide described herein in a method for inhibiting, reducing or preventing abnormal Tau aggregation, the method comprising steps of: contacting abnormal Tau protein, or a biological sample or system containing abnormal Tau protein, with an effective amount of the inhibitory peptide under conditions allowing the inhibitory peptide to bind to the PHF6 sequence present in abnormal Tau so that abnormal Tau aggregation is inhibited, reduced or prevented.

The inhibitory peptide used in such a method may be one of the inhibitory peptides described herein or a combination of at least two of the inhibitory peptides described herein. The method may be carried out *in vitro* (*e.g.,* in cells or other biological system expressing abnormal tau), or *in vivo* (in animals, animal models, or patients). Patient-derived or genome-edited pluripotent stem cells (PSCs) have helped generate valid and robust *in vitro* cell models for tauopathies (Shi et al., Sci. Transl. Med., 2012, 4(124): 124ra29; Iovino et al., Brain, 2015, 138(11): 334563359; Wray, Brain Pathl., 2017, 27(4): 525-529; Arber et al., Alzheimer's Research & Therapy, 2017, 9: 42; Garcia-Leon et al., Alzheimers Dement., 2018, 14(10) : 1261-1280 ; Karch et al., Stem Cell Reports, 2019, 13(5): 939-955; Lin et al., Pharmaceuticals, 2021, 14(6): 525; Penney et al., Molecular Psychiatry, 2020, 25: 148-167). Kim and his coworkers (Kim et al., Nature Protoc., 2015, 10(7): 985-100; Kwak et al., Nature Commun., 2020, 11, 1377, doi.org/10.1038/s41467-020-15120-3) created a three-dimensional (3D) human neural stem cell model of Alzheimer's Disease using β-amyloid precursor protein and presenilin-1 overexpressing ReNcell™ VM human neural stem cell lines. This 3D cell model is able to induce robust extracellular deposition of amyloid-β, including amyloid-β plaques, and high levels of phosphorylated Tau in the soma and neurites, as well as filamentous tau. 3D neural stem cell models of Alzheimer's Disease are also commercially available from Aldrich under the name Alzheimer's In a Dish™. Most of the animal models of tauopathies involve genetically modified animals (mostly rodents, but also fruit fly, zebrafish, and worm). For a review of animal models of tauopathies, see Dujardin et al., Neuropathol. Appl. Neurobiol., 2015, 41(1): 59-80; Combs et al., Methods Mol. Biol., 2016, 1382: 339-366; Cubinkova et al., Acta Virol., 2017, 61(1): 13-21; Götz et al., Adv. Exp. Med. Biol., 2019, 1184: 381-391; Goodarzi et al., Cell Tissue Bank, 2019, 20(2): 141-151; *et al.,* Alzheimers Dement. (NY), 2020, 6(1): e12114; Giong et al., Int. J. Mol. Sci., 2021, 22(16): 8465). When carried out *in vivo,* the contacting step typically includes administering the inhibitory peptide to the animal, animal model or patient (see below). When carried out *in vivo,* the method may be used to inhibit development or progression of a tauopathy or of a disorder associated with abnormal Tau aggregation in an subject. Thus, the method may be used to treat or prevent a tauopathy or a disorder associated with abnormal Tau aggregation in an subject.

Therefore, the present invention also relates to an inhibitory peptide, or combination thereof, (optionally after formulation with one or more appropriate pharmaceutically acceptable carriers or excipient), for use in the treatment or prevention of a tauopathy or a disorder associated with abnormal Tau aggregation in a subject. The present invention also relates to a method for the treatment or prevention of a tauopathy or a disorder associated with abnormal Tau aggregation in a subject, the method comprising a step of administering to the subject in need thereof a therapeutically effective amount of at least one inhibitory peptide, or combination thereof, or a pharmaceutical composition thereof. Another aspect of the invention is a method to prevent the onset of a tauopathy or a disorder associated with abnormal Tau aggregation in a subject, or to treat a subject in the early stages of such diseases or conditions, or a subject that is developing such as disease or condition, in order to prevent or inhibit the development of the disease or condition.

### A. Indications

In many embodiments, the subject is a human being, and more specifically a patient who has been diagnosed with a tauopathy or a subject who has been determined as susceptible to develop a tauopathy.

A tauopathy that can be treated according to a method described herein may be any neurodegenerative disease involving the abnormal aggregation of Tau protein into neurofibrillary or gliofibrially tangles in the human brain. Clinically, tauopathies can present as cognitive syndromes, movement disorders, motor neuron disease, or mixed. A tauopathy that can be treated according to a method described herein include primary tauopathies and secondary tauopathies. The treated tauopathy may be a 3R tauopathy, a 4R tauopathy, or a 3R/4R mixed tauopathy. In particular, a tauopathy that can be treated using a method described herein include Alzheimer's disease (AD), Pick's disease (PD), progressive supranuclear palsy (PSP), corticobasal degeneration (CBD), argyrophilic grain disease (AGD), globular glial tauopathy (GGT), aging-related Tau astrogliopathy (ARTAG), frontotemporal dementia and parkinsonism linked to chromosome 17 (FTDP-17), subacute sclerosing panencephalitis (SSPE), Guadeloupean parkinsonism, Western pacific amyotrophic lateral sclerosis and parkinsonism-dementia complex (ALS/PDC), chronic traumatic encephalopathy (CTE), Huntington Disease (HD); Niemann-Pick disease type C (NPC); Down syndrome; post-encephalitic parkinsonism (PEP), and myotonic dystrophies (types 1 and 2).

In certain particular embodiments, the tauopathy to be treated using a method according to the present invention is selected from the group consisting of Alzheimer's disease, Pick's disease, progressive supranuclear palsy (PSP) and argyrophilic grain disease (AGD). Alzheimer's disease, which is the most prevalent tauopathy, is a progressive disease beginning with mild memory loss and possibly leading to loss of the ability to carry on a conversation and respond to the environment, seriously affecting a person's ability to carry out daily activities. Pick's disease (or frontotemporal dementia (FTD)) is a rare type of dementia that affects the frontal lobes of the brain and causes speech problems like aphasia, behavior difficulties and eventually death. Second only to Alzheimer's in prevalence, Pick' disease accounts for 20% of early-onset dementia cases. It can occur in people as young as 20, but it usually begins between ages 45 and 65, approximately equally affecting men and women. Progressive supranuclear palsy (PSP) is a late-onset degenerative disease involving the gradual deterioration and death of specific volumes of the brain. The condition leads to symptoms including loss of balance, slowing of movement, difficulty moving the eyes, and cognitive impairment. PSP affects about 6 people per 100,000, the first symptoms typically occurring in persons aged 60-70, with men being slightly more likely to be affected than women. Argyrophilic grain disease (AGD) is highly frequent, but still an under-recognized neurodegenerative condition. AGD has proven to be the second-most common neurodegenerative disease after Alzheimer's. The incidence of AGD increases significantly with age and its prevalence has been estimated to range from 9.3% in 65-year olds to 31.3% in centenarians. AGD is clinically characterized by amnesia, with other cognitive functions relatively spared, and prominent neuropsychiatric features.

The effects of a treatment according to the invention may be monitored using any of the assays known in the art for the diagnosis of the disease being treated. Alternatively, or additionally, the effects of a treatment according to the present invention may be monitored using a diagnostic method described herein (see below).

### B. Administration

An inhibitory peptide, or combination thereof (optionally after formulation with one or more appropriate pharmaceutically acceptable carriers or excipients), in a desired dosage can be administered to a subject in need thereof by any suitable route. Various delivery systems are known and can be used to administer inhibitory peptides of the present invention, including tablets, capsules, injectable solutions, encapsulation in liposomes, microparticles, microcapsules, etc. Methods of administration include, but are not limited to, dermal, intradermal, intramuscular, intraperitoneal, intralesional, intravenous, subcutaneous, intranasal, pulmonary, epidural, ocular, and oral routes. An inhibitory peptide, or composition thereof, may be administered by any convenient or other appropriate route, for example, by infusion or bolus injection, by adsorption through epithelial or mucocutaneous linings (*e.g.*, oral, mucosa, rectal and intestinal mucosa, etc). Administration can be systemic or local. In particular, administration may be by injection into CSF (cerebrospinal fluid) pathways, or direct injection into the brain. Parenteral administration may be directed to a given tissue of the patient, such as by catheterization. As will be appreciated by those of ordinary skill in the art, in embodiments where an inhibitory peptide, or composition thereof, is administered along with an additional therapeutic agent, the inhibitory peptide and the therapeutic agent may be administered by the same route (*e.g.,* orally) or by different routes (*e.g.,* orally and intravenously).

An inhibitory peptide, or a combination thereof, (optionally after formulation with one or more appropriate pharmaceutically acceptable carriers or excipients) may alternatively be administered incorporated in catheters, needles, or implants.

*C. Dosage*

Administration of an inhibitory peptide, or combination thereof, (optionally after formulation with one or more appropriate pharmaceutically acceptable) will be in a dosage such that the amount delivered is effective for the intended purpose. The route of administration, formulation and dosage administered will depend upon the therapeutic effect desired, the severity of the disease being treated, the age, sex, weight and general health condition of the patient as well as upon the potency, bioavailability and *in vivo* half-life of the inhibitory peptide, or combination thereof, used, the use (or not) of concomitant therapies, and other clinical factors. These factors are readily determinable by the attending physician in the course of the therapy. Alternatively, or additionally, the dosage to be administered can be determined from studies using animal models. Adjusting the dose to achieve maximal efficacy based on these or other methods are well known in the art and are within the capabilities of trained physicians. As studies are conducted using inhibitory peptides of the invention, further information will emerge regarding the appropriate dosage levels and duration of treatment.

A treatment according to the present invention may consist of a single dose or multiple doses. Thus, administration of an inhibitory peptide, or pharmaceutical composition thereof, may be constant for a certain period of time or periodic and at specific intervals, *e.g.*, hourly, daily, weekly (or at some other multiple day interval), monthly, yearly (*e.g.,* in a time release form). Alternatively, the delivery may occur at multiple times during a given time period, *e.g.,* two or more times per week, two or more times per month, and the like. The delivery may be continuous delivery for a period of time, e.g., intravenous delivery.

In general, however, a suitable dose will be in the range of from about 0.001 to about 100 mg/kg body weight of the recipient per day, *e.g.,* from about 0.01 to about 100 mg/kg of body weight per day, such as above about 0.1 mg/kg body weight per day, or in a range of from about 1 to about 10 mg/kg of body weight per day. For example, a suitable dose can be about 1 mg/kg, 5 mg/kg, 10 mg/kg, 20 mg/kg, or 30 mg/kg of body weight per day.

The inhibitory peptides of the present invention may be conveniently administered in unit dosage form, for example, containing 0.05 to 10000 mg, 0.5 to 10000 mg, 5 to 1000 mg, or about 100 mg of active ingredient per unit dosage form. In some embodiments, the dosage unit contains about 0.1 mg, about 0.5 mg, about 1 mg, about 10 mg, about 25 mg, about 50 mg, about 75 mg, or about 100 mg, of active ingredient.

**D. Concomitant Therapies**

A treatment according to the present invention may be administered alone or in combination with another therapy (*i.e.,* a therapeutic agent and/or a therapeutic procedure), in particular a therapy known to be beneficial to a patient suffering from a tauopathy. The inhibitory peptide, or a combination thereof, (optionally after formulation with one or more appropriate pharmaceutically acceptable carriers or excipients) may be administered prior to administration of the additional therapeutic agent or procedure, concurrently with the therapeutic agent or procedure, and/or following administration of the additional therapeutic agent or procedure.

Treatment of tau-related neurodegenerative syndromes is limited and largely symptomatic. Currently, there are two classes of medication approved for cognition:

cholinesterase inhibitors and an N-methyl-D-aspartate (NMDA) receptor antagonist, each demonstrating modest effects. Motor symptoms may respond to dopaminergic regimens, speech therapy has shown some efficacy with aphasia syndromes (acquired language dysfunctions due to neurological injury or disease), and physical therapy can prove helpful in prolonging motor function. Symptoms of apathy and depression are quite common and often respond to therapy or pharmacologic intervention, including selective serotonin reuptake inhibitors. Thus, an inhibitory peptide or combination thereof, or a pharmaceutical composition thereof, may be administered in combination with at least one additional therapeutic agent or therapeutic procedure selected from: a cholinesterase inhibitor, a N-methyl-D-aspartate (NMDA) receptor antagonist, a dopaminergic regimen, a selective serotonin reuptake inhibitor, speech therapy, physical therapy, and any beneficial combination thereof.

**2 - Diagnostic Applications**

An inhibitory peptide described herein, which specifically interacts with the PHF6 sequence present in Tau protein can be used to detect abnormal or pathological Tau, *i.e.,* Tau which, due to loss of function or deregulation, is found free in the cytoplasm of neurons. In certain preferred embodiments, the inhibitor peptide comprises a detectable moiety.

Thus, the present invention relates to an *in vitro* method for detecting the presence or absence of abnormal Tau protein in a biological sample, the method comprising steps of: contacting the biological sample with an effective amount of an inhibitory peptide, preferably but not necessarily labeled with a detectable moiety, under conditions allowing the inhibitory peptide to bind to abnormal Tau protein; and detecting the presence or absence of an inhibitory peptide bound to abnormal Tau protein in the biological sample. The method may be performed on a biological sample obtained from an individual suspected of suffering from a tauopathy (for example a sample of cerebrospinal fluid). Such a method may be used, for example in diagnostic strategies designed to observe the presence or status of a tauopathy (*e.g.,* Alzheimer's disease or Pick's disease), for example to detect disease beginnings before clinical symptoms, and/or to follow the effectiveness (or lack of effectiveness) of a therapeutic treatment.

The present invention also relates to an inhibitory peptide, or a composition thereof, for use in the diagnosis of a tauopathy or a disorder associated with abnormal Tau aggregation in a subject. The present invention also relates to a method for the diagnosis of a tauopathy or a disorder associated with abnormal Tau aggregation in a subject, the method comprising steps of: administering to the subject a diagnostically effective amount of an inhibitory peptide, or a composition thereof; and detecting any inhibitory peptide bound to abnormal Tau protein, wherein detection of inhibitory peptide bound to abnormal Tau protein indicates that the subject is suffering from, or is at risk of developing, a tauopathy or a disorder associated with abnormal Tau aggregation. In particular, the method may be used for an early diagnosis of a tauopathy or a disorder associated with abnormal Tau aggregation. Alternatively, or additionally, the method may be used to select a therapy specifically adapted to the tested patient.

Administration of a diagnostically effective amount of an inhibitory peptide, or a composition thereof, to the subject may be performed using any suitable administration method known in the art (see above). However, in certain embodiments, administration is preferably carried out by injection of a composition, e.g., an aqueous composition, comprising the labeled inhibitory peptide. The dose of the labeled inhibitory peptide will vary depending on the exact compound to be administered, the weight of the patient, and other variables as would be apparent to a physician skilled in the art. Generally, the dose could preferably lie in the range 0.001 $\mu$g/kg to 10 $\mu$g/kg, preferably 0.01 $\mu$g/kg to 1.0 $\mu$g/kg. When the inhibitory peptide used in a diagnostic method is labeled with a radioactive moiety, the radioactive dose can be such that about 1 $\mu$Ci to about 10 mCi/kg body weight, or about 5 $\mu$Ci to about 1 mCi/kg body weight, or about 10 $\mu$Ci to about 100 $\mu$Ci/kg body weight, or about 1 mCi to about 10 mCi/ kg body weight is administered at a time. The radioactive does may be, for example, 100 to 600 MBq, more preferably 150 to 450 MBq.

As known in the art, after administration and prior to the detection step, the labeled inhibitory peptide is allowed to distribute into the tissue of interest (here brain tissue) and to bind to any free abnormal Tau protein present. The amount of time required for tissue distribution and binding will depend on the labeled inhibitory peptide and can be determined by one skilled in the art by routine experiments.

As will be recognized by one skilled in the art, the inhibitory peptide labeled with a detectable moiety will be selected based on the detection technique intended to be used for the diagnosis. Preferably, the detection is carried out using an imaging technique, and the diagnostic method comprises a step of imaging at least part of the brain of the subject to detect any inhibitory peptide bound to abnormal Tau protein. The most widely used neuroimaging modalities are magnetic resonance imaging (MRI), computerized tomography (CT), positron emission tomography (PET), and single-photon emission computed tomography (SPECT). In certain preferred embodiments, imaging is performed using PET.

In other embodiments, multimodal imaging or multiplexed imaging is used in the detection step. Multimodal imaging or multiplexed imaging refers to simultaneous production of signals from more than one imaging technique. The first main reason is that there exists a great complementarity between different imaging modes. For instance, the images obtained by positron emission tomography imaging (PET) or single-photon emission tomography imaging (SPECT) do not contain high resolution, three-dimensional anatomical information. On the other hand, high-resolution structural images can be obtained *via* the use of CT and/or MRI. These images complement each other to provide a complete picture of the targeted organs' anatomy, physiology, and pathology. Thus, in certain embodiment, the detection or imaging step is carried out by a multimodal imaging technique involving MRI-based imaging and PET-based imaging.

An imaging technique may provide quantitative data. Thus, the amount or level of labeled inhibitory peptide bound to abnormal Tau detected in the brain or part of the brain of the subject may be compared to a normal control amount or level, which has been determined in the brain or the same part of the brain of a healthy subject (*i.e.,* not suffering from a tauopathy or a disorder associated with abnormal Tau aggregation) or to a normal control amount or level, which is a mean value of determinations carried out in several (*e.g.,* 5, 10, 20, 50, 100, 500 or more than 500) healthy subjects. An amount or level of labeled inhibitory peptide bound to abnormal Tau detected in the brain or part of the brain of the subject that is higher than the normal control amount or level indicates that the subject is suffering from, or is at risk of developing, a tauopathy or a disorder associated with abnormal Tau aggregation. The amount or level of labeled inhibitory peptide bound to abnormal Tau detected in the brain or part of the brain of the subject may further be compared to one or more predetermined thresholds corresponding to different diagnoses (early tauopathy, different stages of a tauopathy, a particular tauopathy, etc....).

Using a diagnostic method described herein, skilled physicians may, based on the diagnosis reached, select and prescribe treatments adapted to each patient. Selection of an appropriate therapeutic regimen for a given patient may be made based solely on the diagnosis provided by the diagnostic method disclosed herein. Alternatively, the physician may also consider other clinical or pathological parameters used in existing methods to diagnose a tauopathy.

A diagnostic method described herein may be used to monitor the effectiveness of a tauopathy treatment in a patient. In such a method, the amount or level of labeled inhibitory peptide bound to abnormal Tau may be detected at various points of time during the treatment, for instance, before and after onset of the treatment or at various points of time after the onset of the treatment. A decrease in the amount or level of labeled inhibitory peptide bound to abnormal Tau is indicative that the tauopathy treatment is effective in the patient, or in other words that the patient is responsive to the tauopathy treatment.

## III - Pharmaceutical Compositions and Packs and Diagnostic Kits

### 1 - Pharmaceutical Compositions

As mentioned above, in therapeutic and/or diagnostic applications, an inhibitory peptide described herein, may be administered *per se* or as a pharmaceutical composition. Accordingly, the present invention provides a pharmaceutical composition comprising an effective amount of at least one inhibitory peptide and at least one pharmaceutically acceptable carrier or excipient. In some embodiments, the pharmaceutical composition further comprises one or more additional biologically active agents.

The pharmaceutical compositions of the present invention may be formulated in dosage unit form for ease of administration and uniformity of dosage. The expression "unit dosage form", as used herein, refers to a physically discrete unit of at least one inhibitory peptide for the patient to be treated/subject to be tested. It will be understood, however, that the total daily dosage of the compositions will be decided by the attending physician within the scope of sound medical judgement.

### A. Formulation

A pharmaceutical composition described herein may be administered in any amount and using any route of administration effective for achieving the desired prophylactic and/or therapeutic effect and/or diagnostic goal. The optimal pharmaceutical formulation can be varied depending upon the route of administration (orally, nasally, intraperitoneally, or parenterally, by intravenous, intramuscular, topical, or subcutaneous routes, or by injection into tissue), and desired dosage. Such formulations may influence the physical state, stability, rate of *in vivo* release, and rate of *in vivo* clearance of the administered active ingredient.

Injectable preparations, for example, sterile injectable aqueous or oleaginous suspensions may be formulated according to the known art using suitable dispersing or wetting agents, and suspending agents. The sterile injectable preparation may also be a sterile injectable solution, suspension or emulsion in a non-toxic parenterally acceptable diluent or solvent, for example, as a solution in 2,3-butanediol. Among the acceptable vehicles and solvents that may be employed are water, Ringer's solution, U.S.P. and isotonic sodium chloride solution. In addition, sterile, fixed oils are conventionally employed as a solution or suspending medium. For this purpose, any bland fixed oil can be employed including synthetic mono- or di-glycerides. Fatty acids such as oleic acid may also be used in the preparation of injectable formulations. Sterile liquid carriers are useful in sterile liquid form compositions for parenteral administration.

Injectable formulations can be sterilized, for example, by filtration through a bacterial-retaining filter, or by incorporating sterilizing agents in the form of sterile solid compositions which can be dissolved or dispersed in sterile water or other sterile injectable medium prior to use. Liquid pharmaceutical compositions which are sterile solutions or suspensions can be administered by, for example, intravenous, intramuscular, intraperitoneal or subcutaneous injection. Injection may be *via* single push or by gradual infusion. Where necessary or desired, the composition may include a local anesthetic to ease pain at the site of injection.

In order to prolong the effect of an active ingredient, it is often desirable to slow the absorption of the ingredient from subcutaneous or intramuscular injection. Delaying absorption of a parenterally administered active ingredient may be accomplished by dissolving or suspending the ingredient in an oil vehicle. Injectable depot forms are made by forming micro-encapsulated matrices of the active ingredient in biodegradable polymers such as polylactide-polyglycolide. Depending upon the ratio of active ingredient to polymer and the nature of the particular polymer employed, the rate of ingredient release can be controlled. Examples of other biodegradable polymers include poly(orthoesters) and poly(anhydrides). Depot injectable formulations can also be prepared by entrapping the active ingredient in liposomes or microemulsions which are compatible with body tissues.

Liquid dosage forms for oral administration include, but are not limited to, pharmaceutically acceptable emulsions, microemulsions, solutions, suspensions, syrups, elixirs, and pressurized compositions. In addition to at least one inhibitory peptide, the liquid dosage form may contain inert diluents commonly used in the art such as, for example, water or other solvent, solubilising agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cotton seed, ground nut, corn, germ, olive, castor, and sesame oils), glycerol, tetrahydrofurfuryl alcohol, polyethylene glycols, and fatty acid esters of sorbitan and mixtures thereof. Besides inert diluents, the oral compositions can also include adjuvants such as wetting agents, suspending agents, preservatives, sweetening, flavouring, and perfuming agents, thickening agents, colors, viscosity regulators, stabilizes or osmo-regulators. Examples of suitable liquid carriers for oral administration include water (potentially containing additives as above, e.g., cellulose derivatives, such as sodium carboxymethyl cellulose solution), alcohols (including monohydric alcohols and polyhydric alcohols such as glycols) and their derivatives, and oils (*e.g.*, fractionated coconut oil and arachis oil). For pressurized compositions, the liquid carrier can be halogenated hydrocarbon or other pharmaceutically acceptable propellant.

Solid dosage forms for oral administration include, for example, lozenges, troches, tablets, capsules, effervescent tablets, orally disintegrating tablets, floating tablets designed to increase gastric retention times, buccal patches and sublingual tablets. In such solid dosage forms, an inhibitory peptide, or a combination thereof, may be mixed with at least one inert, pharmaceutically acceptable excipient or carrier such as sodium citrate or dicalcium phosphate and one or more of: (a) fillers or extenders such as starches, lactose, sucrose, glucose, mannital, and silicic acid; (b) binders such as, for example, carboxymethylcellulose, alginates, gelatine, polyvinylpyrrolidone, sucrose, and acacia; (c) humectants such as glycerol; (d) disintegrating agents such as agar-agar, calcium carbonate, potato or tapioca starch, alginic acid, certain silicates, and sodium carbonate; (e) solution retarding agents such as paraffin; absorption accelerators such as quaternary ammonium compounds; (g) wetting agents such as, for example, cetyl alcohol and glycerol monostearate; (h) absorbents such as kaolin and bentonite clay; and (i) lubricants such as talc, calcium stearate, magnesium stearate, solid polyethylene glycols, sodium lauryl sulphate, and mixtures thereof. Other excipients suitable for solid formulations include surface modifying agents such as non-ionic and anionic surface modifying agents. Representative examples of surface modifying agents include, but are not limited to, poloxamer 188, benzalkonium chloride, calcium stearate, cetostearyl alcohol, cetomacrogol emulsifying wax, sorbitan esters, colloidal silicon dioxide, phosphates, sodium dodecylsulfate, magnesium aluminum silicate, and triethanolamine. In the case of capsules, tablets and pills, the dosage form may also comprise buffering agents.

Solid compositions of a similar type may also be employed as fillers in soft and hard-filled gelatine capsules using such excipients as lactose or milk sugar as well as high molecular weight polyethylene glycols and the like. The solid dosage forms of tablets, dragees, capsules, pills, and granules can be prepared with coatings and shells such as enteric coatings, release controlling coatings and other coatings well known in the pharmaceutical formulating art. Examples of embedding compositions which can be used include polymeric substances and waxes.

In certain embodiments, it may be desirable to administer an inventive composition locally. This may be achieved, for example, and not by way of limitation, by local infusion during surgery, topical application, by injection, by means of a catheter, by means of suppository, or by means of a skin patch or stent or other implant.

For topical administration, the composition is preferably formulated as a gel, an ointment, a lotion, or a cream which can include carriers such as water, glycerol, alcohol, propylene glycol, fatty alcohols, triglycerides, fatty acid esters, or mineral oil. Other topical carriers include liquid petroleum, isopropyl palmitate, polyethylene glycol, ethanol (95%), polyoxyethylenemonolaurat (5%) in water, or sodium lauryl sulphate (5%) in water. Other materials such as antioxidants, humectants, viscosity stabilizers, and similar agents may be added as necessary.

In addition, in certain instances, it is expected that the inventive compositions may be disposed within transdermal devices placed upon, in, or under the skin. Such devices include patches, implants, and injections which release the active ingredient by either passive or active release mechanisms. Transdermal administrations include all administration across the surface of the body and the inner linings of bodily passage including epithelial and mucosal tissues. Such administrations may be carried out using the present compositions in lotions, creams, foams, patches, suspensions, solutions, sprays, and suppositories (rectal and vaginal).

Transdermal administration may be accomplished through the use of a transdermal patch containing an active ingredient (*i.e.,* an inhibitory peptide) and a carrier that is non-toxic to the skin, and allows the delivery of the ingredient for systemic absorption into the bloodstream *via* the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may be suitable. A variety of occlusive devices may be used to release the active ingredient into the bloodstream such as a semi-permeable membrane covering a reservoir containing the active ingredient with or without a carrier, or a matrix containing the active ingredient.

Suppository formulations may be made from traditional materials, including cocoa butter, with or without the addition of waxes to alter the suppository's melting point, and glycerine. Water soluble suppository bases, such as polyethylene glycols of various molecular weights, may also be used.

For administration by intranasal delivery (*e.g.*, nasal mucosal delivery of intranasal mucosal delivery) by inhalation or insufflation, at least one inhibitory peptide described herein may be combined with a fine inert powdered carrier or with a liquid carrier. One or more mucosal delivery-enhancing agents may also be present. Examples of absorption enhancers for the intranasal adsorption of peptide drugs include, but are not limited to, bile salts, surfactants, fluidic acid derivatives, phosphatidylcholines, cyclodextrins, and cell-penetrating peptides (CPPs).

Materials and methods for producing various formulations are known in the art and may be adapted for practicing the subject invention. Suitable formulations for the delivery of antibodies can be found, for example, in "Remington's Pharmaceutical Sciences", E.W. Martin, 18th Ed., 1990, Mack Publishing Co.: Easton, PA.

### B. Additional Biologically Active Agents

In certain embodiments, an inhibitory peptide described herein is the only active ingredient in a pharmaceutical composition of the present invention. In other embodiments, the pharmaceutical composition further comprises one or more biologically active agents. Examples of suitable biologically active agents include, but are not limited to, anti-inflammatory agents, immunomodulatory agents, analgesics, antimicrobial agents, antibacterial agents, antibiotics, antioxidants, antiseptic agents, and combinations thereof.

In such pharmaceutical compositions, the inhibitory peptide(s) and the at least one additional therapeutic agent may be combined in one or more preparations for simultaneous, separate or sequential administration of the peptide and therapeutic agent(s). More specifically, an inventive composition may be formulated in such a way that the inhibitory peptide(s) and therapeutic agent(s) can be administered together or independently from each other. For example, the inhibitory peptide(s) and therapeutic agent can be formulated together in a single pharmaceutical composition. Alternatively, they may be maintained (*e.g.*, in different compositions and/or containers) and administered separately, thereby constituting a pharmaceutical kit or pack.

### 2 - Pharmaceutical Packs and Diagnostic Kits

In another aspect, the present invention provides a pharmaceutical pack or kit comprising one or more containers (*e.g.*, vials, ampoules, test tubes, flasks or bottles) containing one or more ingredients of an inventive pharmaceutical composition, allowing administration of at least one inhibitory peptide described herein, to a subject in need thereof, for therapeutic and/or diagnostic purpose.

Different ingredients of a pharmaceutical pack or kit may be supplied in a solid (*e.g.,* lyophilized) or liquid form. Each ingredient will generally be suitable as aliquoted in its respective container or provided in a concentrated form. Packs or kits according to the invention may include media for the reconstitution of lyophilized ingredients. Individual containers of the kits will preferably be maintained in close confinement for commercial sale.

In certain embodiment, a pharmaceutical pack or diagnostic kit can include a device for administering an inhibitory peptide, or composition thereof, e.g., syringe needle, pen device, jet injector or another needle-free injector.

In certain embodiments, a pharmaceutical pack or diagnostic kit includes one or more additional therapeutic agent(s). Optionally associated with the container(s) can be a notice or package insert in the form prescribed by a governmental agency regulating the manufacture, use or sale of pharmaceutical or biological products, which notice reflects approval by the agency of manufacture, use or sale for human administration. The notice of package insert may contain instructions for use of a pharmaceutical composition according to methods of treatment disclosed herein. Alternatively, or additionally, the package insert may contain instructions for use of a diagnostic kit according to a method of diagnostic disclosed herein. Instructions for using the kit according to a diagnostic method may comprise instructions to prepare the patient for administration and/or imaging, and/or instructions for carrying out the imaging part of the diagnostic method, and/or instructions for interpreting the results obtained. A kit may also contain a software package enabling statistical analysis through necessary correction and normalization.

Optionally associated with the container(s) can be a notice or package insert in the form prescribed by a governmental agency regulating the manufacture, use or sale of pharmaceutical or biological products, which notice reflects approval by the agency of manufacture, use or sale for human administration.

An identifier, *e.g.,* a bar code, radio frequency, ID tags, etc., may be present in or on the kit. The identifier can be used, for example, to uniquely identify the kit for purposes of quality control, inventory control, tracking movement between workstations, etc.

The invention will be further illustrated by the following figures and examples. However, these examples and figures should not be interpreted in any way as limiting the scope of the present invention.

## Examples

The following examples describe some of the preferred modes of making and practicing the present invention. However, it should be understood that the examples are for illustrative purposes only and are not meant to limit the scope of the invention. Furthermore, unless the description in an Example is presented in the past tense, the text, like the rest of the specification, is not intended to suggest that experiments were actually carried out or data were actually obtained.

### Identification of Hexapeptides Efficiently Targeting PHF6 Interactions

Two problems remain regarding the management of Alzheimer's disease and other tauopathies. First, the diagnosis is difficult and is often not definitive until postmortem. Second, there is still no treatment that can stop or even slow down pathologies progression. The present study provides new molecules allowing a dual diagnostic/therapeutic approach (theragnostics). More specifically, the goal is to produce, in a single tool, molecules that can be used (i) as a therapeutic agent for the treatment of tauopathies, and (ii) as a probe for the early diagnosis of tauopathies. The basic backbone of these molecules is a synthetic hexapeptide which inhibits intramolecular interactions involved in the formation of amyloid structures within the abnormal Tau protein. More precisely, the identified hexapeptides target the interactions of the PHF6 peptide ([306]VQIVYK[311], SEQ ID NO: 2) with itself and with [374]HKLTF[378] (SEQ ID NO: 3) which are observed in the case of Alzheimer's disease and the interactions of the PHF6 peptide with itself and with [336]QVEVKS[341] (SEQ ID NO: 4) which are observed in the case of Pick's disease. These interactions, which are responsible for the nucleation processes of amyloid aggregations, occur through two distinct or simultaneous nucleation steps, *i.e.,* $\beta1$-$\beta1$ and $\beta1$-$\beta8$ in Alzheimer's disease and $\beta1$-$\beta1$ and $\beta1$-$\beta4$ in Pick's disease (see Figure 1).

### I. Selection of Synthetic Hexapeptides Derived from VQIVYK (SEQ ID NO: 2)

### A. Hexapeptides Studied

### 1. First 22 Hexapeptides

In a previous study aimed at providing answers regarding the laws governing amyloid aggregation (Thesis "Chaperons moléculaires et tauophathies: effet de Hsp90 sur la fibrillation in vitro du peptide VQIVYK issu de la protéine tau", Claire Schirmer, 2014, Université de Rennes, France), the research group of the present inventors carried out a comparative study of the amyloidogenicity of permutation variants derived from the VQIVYK peptide. Swapping each of the 6 amino acids (2 Valine residues, 1 Glutamine, 1 Isoleucine, 1 Tyrosine and 1 Lysine) gives 360 possible combinations. Among these 360 combinations, only 93 are actually found in proteins. Interestingly, all 360 variants have aggregation scores between 67.592 and 93.416. These scores are particularly high, since of the 64 million possible hexapeptides, only 219387 of them (0.34%) have a score greater than or equal to 67.592. Twenty-two (22) sequences were selected based on their "MetAmyl" score compared to that of the reference peptide VQIVYK (score of 67,671) derived from the Tau protein (Table 2).

**Table 2.** Names and Sequences of VQIVYK and the 22 derived peptides.

| Peptide | Sequence | Found in human proteins ? | SEQ ID NO: |
|---------|----------|---------------------------|------------|
| 1p | VQIVYK | yes | SEQ ID NO: 29 |
| 2p | KYIQVV | yes | SEQ ID NO: 30 |
| 3p | VKYQVI | yes | SEQ ID NO: 5 |
| 4p | VQKIYV | yes | SEQ ID NO: 6 |

(continued)

| Peptide | Sequence | Found in human proteins ? | SEQ ID NO: |
|---------|----------|---------------------------|------------|
| 5p | IKQVYV | ves | SEQ ID NO: 7 |
| 6p | VVQKYI | yes | SEQ ID NO: 8 |
| 7p | VKVIYQ | yes | SEQ ID NO: 31 |
| 8p | QKYIVV | no | SEQ ID NO: 9 |
| 9p | IYVQKV | yes | SEQ ID NO: 32 |
| 10p | KVQIVY | no | SEQ ID NO: 33 |
| 11p | VKVYIQ | yes | SEQ ID NO: 34 |
| 12p | VQVIYK | no | SEQ ID NO: 35 |
| 13p | YVVQK | no | SEQ ID NO: 11 |
| 14p | KVQYVI | no | SEQ ID NO: 12 |
| 15p | KYVIQV | no | SEQ ID NO: 36 |
| 16p | YQVKIV | no | SEQ ID NO: 37 |
| 17p | YQVIVK | ves | SEQ ID NO: 38 |
| 18p | QKVVYI | yes | SEQ ID NO: 39 |
| 19p | QKVYIV | no | SEQ ID NO: 40 |
| 20p | KQIVYV | no | SEQ ID NO: 41 |
| 21p | VQVKYI | no | SEQ ID NO: 42 |
| 22p | VQVYKI | no | SEQ ID NO: 43 |
| 23p | QVIKYV | yes | SEQ ID NO: 13 |

**2. Additional 10 Hexapeptides**

As previously described VQIVYK (peptide **1p**) is a segment essential for the formation of amyloid fibers for the entire Tau protein. In addition, it constitutes a good model for the study of the association process *in vitro,* the structures formed *in vitro* are parallel β sheets which interact through their side chains in a "face-to-face" and "back-to-up" fashion. To compete with the "face-to-face" interaction, a potential inhibitory peptide should have at least the Glutamine-Valine-Lysine (QVK) or Valine-Isoleucine-Tyrosine (VIY) sequences on either side of the peptidic chain and in the same positions as VQIVYK. Among the 22 peptides described above and previously studied, none show such a sequence. Therefore, 10 additional hexapeptides (see Table 3) were designed and produced - these 10 hexapeptides have the particularity of exhibiting the 3 even or odd amino acids at a position identical to V̲Q̲I̲V̲Y̲K̲ (*i.e.,* V̲ - I̲ - Y̲, or **Q** - **V** - **K**) to be able to enter in competition with the "face-to-face" interaction observed for V̲Q̲I̲V̲Y̲K̲.

**Table 3.** Names and Sequences of the 10 shuffled hexapeptides of the PHF6 peptide.

| Peptide | Sequence | Found in human proteins ? | SEQ ID NO: |
|---------|----------|---------------------------|------------|
| 0tp | V̲Q̲I̲K̲Y̲V̲ | no | SEQ ID NO: 44 |
| 1tp | V̲K̲I̲V̲Y̲Q̲ | yes | SEQ ID NO: 14 |
| 2tp | V̲V̲I̲Q̲Y̲K̲ | no | SEQ ID NO: 45 |
| 3tp | V̲V̲I̲K̲Y̲Q̲ | no | SEQ ID NO: 46 |
| 4tp | V̲K̲I̲Q̲Y̲V̲ | no | SEQ ID NO: 15 |
| 5tp | V**Q**Y**VI**K | no | SEQ ID NO: 47 |
| 6tp | I**Q**VV**V**K | no | SEQ ID NO: 16 |
| 7tp | I**Q**VVY**K** | no | SEQ ID NO: 48 |

(continued)

| Peptide | Sequence | Found in human proteins ? | SEQ ID NO: |
|---------|----------|---------------------------|------------|
| 8tp | IQYVVK | no | SEQ ID NO: 49 |
| 9tp | YQVVIK | no | SEQ ID NO: 50 |

The 10 peptides were studied in the same way as the previous 22 peptides, both by bioinformatics and by biochemical techniques.

## B. Materials and Methods

### 1. Solubilization of Peptides

The PHF6 peptide (Ac-VQIVYK-NH2) as well as the derived hexapeptides were produced by Proteogenix® in an N-acetylated and C-amidated form and supplied in a lyophilized form. They are acetylated and amidated in order to better mimic the original protein. They were resuspended in milli Q water and filtered through 0.2 $\mu$m to obtain a concentration close to 5 mg/mL. After being vortexed for 15 minutes, the peptide solution was sonicated for 15 minutes at 35°C in a sonicator water bath (Advantage-Lab AL04-04) then ultracentrifuged (Beckman TL100) at 100,000g for 10 minutes at 20°C. The pellet containing the residual aggregates was discarded. The concentration of the supernatant was determined by measuring UV absorption at 280 nm using a molar extinction coefficient of 1450 $M^{-1}cm^{-1}$ in 1% SDS.

### 2. Polymerization of Peptides

After thawing, a pre-dilution in milli Q $H_2O$ filtered through 0.2 $\mu$m was carried out in order to obtain a solution at a concentration close to the working concentration, greater than 1.25 times. This stock solution was left to incubate for 1 hour in a water bath at 25°C, and was then assayed spectrophotometrically and adjusted to the final working concentration of 1.25 times. The polymerization was initiated by adding 1/4 of the volume of 5X fibrillation buffer (50 mM MOPS, 750 mM NaCl, pH = 7.2) for a final concentration of 1X fibrillation buffer (10 mM MOPS, 150 mM NaCl, PH = 7.2). The samples were left to incubate for 1.5-2 hours at 25°C in a water bath. The study of the polymerization by fluorimetry was carried out under the same conditions, except that the 5X polymerization buffer was supplemented with Thioflavin T (ThT) at 100 $\mu$M in order to be able to observe the fluorescence.

*(a) Determination of the Aggregative Character of the Peptides.* Once polymerized, the samples were ultracentrifuged at 100,000g for 5 minutes at 25°C (Beckman TL100, rotor TL-A100). The supernatants of each sample were then collected. An assay at 280 nm in triplicate of each supernatant was then carried out in the presence of 1% SDS. The concentration of the supernatant was determined using the Beer-Lambert law A = $\varepsilon$ x 1 x C; $\varepsilon$ (peptides) = 1450 $M^{-1}cm^{-1}$. The concentration of sedimentable species in the pellet was then deduced by the difference: "total concentration of the sample - concentration of free peptide present in the supernatant".

*(b) Determination of the Amyloidogenic Character of the Peptides.* Once the peptides have polymerized as previously indicated, 40 $\mu$L of peptides polymerized at a concentration of 400 $\mu$M and 800 $\mu$M were diluted in 360 $\mu$E of Thioflavin T (10 $\mu$M ThT, 1X MOPS) in a thermostatically controlled tank of 0.2x1 cm, to a final volume of 400 $\mu$L. The fluorescence of the sample was then measured immediately after dilution by recording a fluorescence spectrum (470 nm to 600 nm) at an excitation wavelength of 450 nm (Perkin Elmer LS 55, PM 750, slit10).

### 4. Bioinformatic Prediction of the Amyloidogenic Character of the Peptides.

The prediction of the amyloidogenicity of the peptides was carried out on a virtual sequence composed of the various peptides concatenated and separated from each other by a "spacer" peptide, flanked by two glycine residus, known to have a zero aggregative character, GPNSKQSQDEG (SEQ ID NO: 51). Four independent prediction methods (Aggrescan, Pasta 2.0, Tango and Salsa) were used, as described by Garnier et al. (Sci. Rep., 2017, 7: 6812). The results obtained with the Aggrescan and Salsa prediction methods were found to be not discriminating, these two methods were not taken into account and only the Tango and Pasta 2.0 methods were used. For each of these two methods, a score of 100% was assigned to the amino acid with the highest probability. Each amino acid was assigned the average of the percentages obtained using the two different methods. The score for each peptide is the average of the scores for each amino acid that constitutes it. All the scores for each peptide were normalized considering that the reference peptide VQIVYK (**1p**) had a score of 100%.

## C. Results

### 1. Bioinformatic Approach

The results obtained are presented on Figure 2. The amyloidogenicity score of **1p** was set at 100% and the score of the other peptides was normalized according to **1p** . In the set of the 22 peptides (in red in Figure 2), the percentage of amyloidogenicity thus calculated was found to vary from 3 for the **6p** peptide to 160 for the **13p** peptide; and the 22 hexapeptides can be grouped into 3 categories: those predicted to be strongly amyloidogenic **(1p, 12p, 13p, and 17p),** weakly amyloidogenic **(3p, 4p, 5p, 6p, 9p, 21p, 22p,** and **23p),** and peptides in between. In the set of the 10 additional peptides (in green in Figure 2), the percentage of amyloidogenicity was found to vary from 9 for the **0tp** peptide to 100 for the **2tp** peptide; and the 10 hexapeptides can be grouped into 2 categories: those predicted to be strongly amyloidogenic **(1tp, 2tp, 3tp, 5tp, 6tp, 7tp, 8tp** et **9tp)** and weakly amyloidogenic **(0tp, 4tp** et **10tp).** Due to the chain of amino acids, this batch of 10 additional hexapeptides is therefore mainly amyloidogenic.

### 2. Biochemical Approach

After prediction of their amyloidogenic character by bioinformatics, all the peptides were studied using biochemical techniques, namely sedimentation and fluorescence/ThT. The biochemical experiments were carried out at two peptide concentrations: 400 $\mu$M and 800 $\mu$M. Only the results obtained at 800 $\mu$M are presented. At this concentration, in sedimentation, the amount of sedimentable peptide after polymerization was measured and expressed as a percentage as a function of the total concentration. This value corresponds to the aggregative character of the peptides. On the other hand, the fluorescence test was used to determine their amyloidogenic character, the fluorescence value given corresponds to a normalization with respect to **1p,** considering that its fluorescence is 100% (Table 4).

**Table 4.** Results of the biochemical experiments carried out by sedimentation and fluorescence.

| Peptide | % sedimentation | % fluorescence | Peptide | % sedimentation | % fluorescence |
|---|---|---|---|---|---|
| **1p** | 94 | 100 | **0tp** | 0 | 10.7 |
| **2p** | 67 | 2.8 | **1tp** | 0 | 0.9 |
| **3p** | 18 | 0.2 | **2tp** | 58 | 2.9 |
| **4p** | 1 | 0.8 | **3tp** | 48 | 0.9 |
| **5p** | 5 | 1.1 | **4tp** | 0 | 2 |
| **6p** | 14 | 0.2 | **5tp** | 26 | 71.7 |
| **7p** | 86 | 1.3 | **6tp** | 0 | 1.3 |

(continued)

| Peptide | % sedimentation | % fluorescence | Peptide | % sedimentation | % fluorescence |
|---|---|---|---|---|---|
| 8p | 0 | 1.9 | 7tp | 77 | 453.6 |
| 9p | 1 | 2.1 | 8tp | 78 | 17.5 |
| 10p | 63 | 3.2 | 9tp | 86 | 7.9 |
| 11p | 6 | 25 | | | |
| 12p | 14 | 81.6 | | | |
| 13p | 0 | 2.7 | | | |
| 14p | 1 | 4.4 | | | |
| 15p | 71 | 3.9 | | | |
| 16p | 94 | 1.7 | | | |
| 17p | 95 | 2.3 | | | |
| 18p | 51 | 6.8 | | | |
| 19p | 76 | 0.8 | | | |
| 20p | 75 | 1.3 | | | |
| 21p | 33 | 19.9 | | | |
| 22p | 34 | 3 | | | |
| 23p | 5 | 0.8 | | | |

As observed in bioinformatics prediction, all the biochemical results show that the peptides exhibit great heterogeneity both in terms of aggregation and amyloidogenic character. In the set of the first 22 hexapeptides, the percentage of sedimentation of the peptides varies from 0 **(8p, 13p)** to ~ 95 (**1p, 17p).** All the peptides studied have a fluorescence lower than that of **1p.** Some have significant fluorescence (82% and 25% for **12p** and **11p** respectively). However, most of them show very weak fluorescence compared to **1p.** In the set of the 10 additional hexapeptides, the percentage of sedimentation of the peptides varies from 0 **(0tp, 1tp** and **4tp)** to -85 **(7tp).** In fluorescence, the **7tp** hexapeptide exhibits a much greater percentage than **1p** (x4.5). Other peptides show significant fluorescence reflecting their amyloidogenic character, with -70% and -20% for **5tp** and **8tp** respectively. The other peptides show very low fluorescence compared to **1p.**

In order to compare the two biochemical parameters studied, the results obtained were presented in the form of a graphic representation of Garnier-Delamarche (Figure 3). In this representation, each peptide is represented as a dot, whose coordinates reflect both its amyloidogenic character and its aggregative character. The **1p** peptide is at the top left of the graph, reflecting its strong amyloidogenic and self-aggregative potential. Among the 22 first peptides, some peptides are amyloidogenic and poorly aggregative **(21p)** and vice versa **(19p).** In the set of the 10 additional hexapeptides, some are amyloidogenic and aggregative (5tp, 7tp) and other are weakly amyloidogenic and weakly aggregative **(1tp, 4tp,** and **6tp).**

Among the 32 hexapeptides studied, 10 were selected for further investigation. These 10 hexapeptides, which are weakly amyloidogenic and weakly aggregative are **4p, 5p, 6p, 9p, 13p, 14p, 23p, 1tp, 4tp,** and **6tp.**

## II. Effect of the 10 Selected Synthetic Hexapeptides on PHF6 polymerization

The study model allowing to observe and characterize the polymerization kinetics of amyloid fibers formed by VQIVYK (PHF6) *in vitro* was first readapted and optimized. The 10 selected hexapeptides derived from VQIVYK **(4p, 5p, 6p, 9p, 13p, 14p, 23p, 1tp, 4tp,** and **6tp)** were then tested for their effects on VQIVYK aggregation.

**A. Materials and Methods**

**1. Solubilization of Peptides.** See above

**2. Preparation of Fibrillation Buffer 5X**

For 100 mL of solution of fibrillation buffer 5X (50 mM MOPS, 750 nM NaCl, pH 7.2): 10 mL of 500 mM MOPS and 25 mL of 3 M NaCl were supplemented with sterile water. The pH was adjusted to 7.2 with sodium hydroxide.

**3. Polymerization of Peptides**

After thawing, a pre-dilution of the peptides of interest in sterile water was carried out in order to obtain, for each peptide, a solution at a concentration close to the working concentration. The peptides were then left to incubate at 4°C overnight. On the day of the experiment, the peptide solutions were sonicated for 10 minutes, left to stand for one hour at room temperature, sonicated again for 10 minutes and assayed spectrophotometrically at 280 nm ($\varepsilon$ = 1450 $M^{-1}cm^{-1}$). The UV spectra were previously normalized to 0 at 300 nm. The solutions were prepared at concentrations of peptides 1/4 above that desired in the end. The polymerization of VQIVYK was initiated by adding 1/4 of the volume of fibrillation buffer 5X for a final concentration of fibrillation buffer 1X (10 mM MOPS, 150 mM NaCl, pH = 7.2).

**4. Critical Assembly Concentration (CCa)**

The polymerization of VQIVYK was initiated by mixing the VQIVYK peptide (at various concentrations, 52 $\mu$M to 344 $\mu$M) with 1/4 of its volume in fibrillation buffer 5X. Once polymerized, the samples were sedimented at 16,900 g for 15 minutes. The concentration of free peptide was measured by assay nanodrop 800. The concentration of polymerized peptide was thus determined using the following the relation:

$$[\mathbf{1p}]_{pellet} = [\mathbf{1p}]_{initial} - [\mathbf{1p}]_{supernatant}$$

**5. Effects of the Derived Peptides on VQIVYK Polymerization by Spectrofluorimetry**

The analyses were carried out on a spectrofluorometer (LS 55 from Perkin Elmer) fitted with a 4-cell rack. Black quartz cells (0.2x1.0 cm) were used. Excitation and emission wavelengths were 450 nm and 480 nm, respectively, and the sensitivity (Photomultiplier "PM") was set at 800 V. The size of the slits was varied from 3 nm to 6 nm. Normalization of each kinetics was performed with respect to the position of the cell on the rack.

**6. Processing of Kinetics Data**

The results were first smoothed over 10 points with the "smooth" option of the KaleidaGraph software. After normalization with respect to the position of the cells in the rack, each start of kinetics was adjusted to 0 before the addition of the fibrillation buffer. Finally, the results of each kinetics were normalized relative to the fluorescence intensity of the **1p** control after 100 minutes of polymerization considered to be 100%.

**B. Results**

**1. Determination of the Critical Assembly Concentration**

As known in the art, it is necessary to determine the Critical Assembly Concentration for each batch of the VQIVYK peptide. The CCa of the batch used in the present study was experimentally determined to be 32 $\mu$M.

**2. Optimization of Spectrofluorimetry Experiments**

It was determined that, for Thioflavin-T (ThT) (an intercalator which allows the study of peptide polymerization), the wavelength values at which maximum fluorescence intensity is observed for excitation and emission are respectively 450 nm and 480 nm. These values were therefore used in the rest of the experiments. The working concentration of ThT was set at 32 $\mu$M, a concentration at which ThT does not influence the rate of polymerization. The concentration of **1p** was set at 155 $\mu$M, for which satisfactory kinetics were obtained (data not shown).

**3. Effects of the 10 Selected Peptides on the Polymerization of PHF6**

As a first approach, it was chosen to work with peptide concentrations twice as high as that of 1p, *i.e.,* at peptide concentrations of 310 mM. Each of the 10 selected peptides was tested only with 1p (Figure 4).

The kinetics of **1p** (red) begins with a nucleation phase followed by a rapid polymerization (elongation) phase and tends towards a saturation phase which forms a plateau. The kinetics of **1p** + **4p** (blue) resembles the kinetics of **1p** alone, with a slower polymerization rate (50% of **1p** alone). The saturation plateau is reached with a fluorescence intensity equal to 80% of that of **1p** alone. Therefore, **4p** inhibits the polymerization of **1p**. In the presence of **23p** (green), a longer nucleation phase is observed, which means that the highest speed is reached later compared to **1p** alone. Although the speed is lower (65% of the speed of **1p** alone), the fluorescence intensity at 100 minutes is higher (161.7% of **1p** alone) and it is likely that a higher plateau is reached later. Since **23p** appears to associate with **1p** to form amyloid structures, it was classified as a non-inhibitory peptide. In the presence of 1tp (orange), it is difficult to distinguish the nucleation phase. During the polymerization phase, the speed is strongly inhibited (12% of the speed of **1p** alone). Likewise, the fluorescence intensity at 100 minutes is very low (23.5% of **1p**). **1tp** is therefore an inhibitory peptide.

By way of example, the kinetics of the three peptides **1tp**, **4tp** and **6tp** in the presence or absence of **1p** are presented on Figure 5. Although not shown, **4p** and 5p are also of therapeutic interest.

The 3 peptides **(1tp, 4tp** and **6tp)** do not polymerize on their own as evidenced by their almost zero fluorescence. **1p** in the presence of **1tp** or of **6tp** was found to exhibit a maximum rate of polymerization and a final fluorescence intensity which are clearly inhibited. Polymerization was followed for 15 hours for these 3 peptides (not shown). Over longer times, it was found that there was no significant change, and that the inhibitory effect persisted.

After having processed and analyzed all the kinetics in the presence of the different peptides, a comparison was made between the fluorescence intensities and the maximum polymerization rates of all the selected peptides (Figure 6). The effect of the different peptides was quantified and the peptides were classified. A threshold of inhibition was set at 50% for the polymerization rate, and for the fluorescence intensity, the threshold was set at 100%.

On the basis of these criteria, out of the 10 selected peptides tested, 5 peptides exhibit a marked inhibitory effect: **4p, 5p, 1tp, 4tp** and **6tp.** The kinetics of polymerization of **1p** in the presence of selected peptides can be classified into 3 categories: kinetics with very high rate and intensity of fluorescence, observed for the **8p** and **23p** peptides; the polymerization kinetics which generally follow the kinetics of **1p** alone such as those observed for the peptides **4p, 5p, 9p, 13p** and **14p,** and the kinetics which are slow compared to **1p** alone and which have a lower fluorescence intensity, such as those observed for the **1tp, 4tp** and **6tp** peptides.

The peptides which were found to show the most interesting inhibition properties were tested several times in order to study their variations (Figure 7).

The inhibitory activity of the selected hexapeptides was also studied *in silico* by molecular modeling using the raptorX program. Figure 8(A) shows the particular example of the effect of the **1tp** hexapeptide on the PHF6-HKLTF interaction. Still using the RaptorX software, it was possible to model *in silico* the interactions occurring within paired helical filaments (PHFs) between the PHF6 peptide (SEQ ID NO: 2) with itself as well as with [374]HKLTF[378] (SEQ ID NO: 3) in the case of Alzheimer's disease (Figure 8(B-I)). It was also possible to test the *in silico* effect of some of the inhibitor peptides **(6tp** and **4tp)** on this structure at different stoichiometries compared to the number of PHF6/HKLTF pairs: stoichiometries of 0,25 (Figure 8(B-II)); 0,50 (Figure 8(B-III)); 0,75 (Figure 8(B-IV)) and 1,00 (Figure 8(B-V)). Figure 9 presents the *in silico* prediction of the interaction of **4tp** with free Tau protein (R3/R4 repeats) and with the PHF structures. This figure clearly shows that **4tp** is able to specifically interact first with free Tau protein, *i.e.* its segments β1 (PHF6, [306]VQIVYK[311]), β4 ([336]QEVVKS[342]), β7 ([356]SLDNITHV[363]) and β8 ([371]IETHKLTF[378]) strands, and second with PHF's extremities. These results show that the **4tp** peptide is able of inhibiting the self-association process of free Tau protein and also of blocking the growth of PHFs.

**C. Discussion**

The 10 selected peptides were classified into 3 categories: peptides "without any inhibitory effect" with the **8p, 9p, 13p, 14p** and **23p** peptides; peptides "with a weak inhibitory effect" with the **4p, 5p** and **4tp** peptides; and peptides "with a strong inhibitory effect" with the **1tp** and **6tp** peptides. Among the 5 selected peptides **(4p, 5p, 1tp, 4tp,** and **6tp)** that are the most interesting in terms of inhibition of PHF6 aggregation, 3 of them **(1tp, 4tp, 6tp)** have a sequence with 3 amino acids at the same position as **1p**.

Future experiments include the study of 10 hexapeptides having the reversed sequence of the 10 selected hexapeptides. In addition, even though they are more aggregative than the 10 selected hexapeptides, it would also be interesting to test peptides 3p and 6p, which are very poorly amyloidogenic. In the same vein, it will be necessary to test the effect of a mixture of hexapeptides on the aggregation of the PHF6 peptide. The VQIVYK/HKLTF interaction model, which corresponds to the true interacting peptides within the human pathological Tau protein β1 for VQIVYK and β8 for HKLTF, will be developed and characterized; and hexapeptides will be tested in this model. *In vitro* tests will be performed using a cell model expressing the pathological human Tau protein; and will be followed by experiments in an model which expresses pathological human tau, in particular the zebra-fish model developed at the ISERT (Institut de Recherche en Santé, Environnement et Travail) in Rennes (France). At the same time, chemical modifications of the most efficient hexapeptides will be undertaken to make them useful in diagnostic methods, and to improve their stability and other properties desirable for therapeutic use in humans.

# EP 4 186 918 A1

**Claims**

1. An inhibitory peptide for use in the diagnostic and/or treatment of a tauopathy or a disorder associated with abnormal Tau aggregation in a subject, wherein the inhibitory peptide consists of, or comprises, a hexapeptide or a derivative thereof, wherein:

   - the hexapeptide consists of an amino acid sequence selected from the group consisting of SEQ ID NO: 6 **(4p),** SEQ ID NO: 18 **(4p$_{inv}$)**,SEQ ID NO: 7 **(5p)**, SEQ ID NO: 19 (**5p$_{inv}$**), SEQ ID NO: 9 **(8p)**, SEQ ID NO: 21 **(8p$_{inv}$)**, SEQ ID NO: 10 **(9p)**, SEQ ID NO: 22 **(9p$_{inv}$)**, SEQ ID NO: 11 **(13p)**, SEQ ID NO: 23 **(13p$_{inv}$)**, SEQ ID NO: 12 **(14p)**, SEQ ID NO: 24 **(14p$_{inv}$)**, SEQ ID NO: 13 **(23p)**, SEQ ID NO: 25 **(23p$_{inv}$)**, SEQ ID NO: 14 (**1tp**), SEQ ID NO: 26 (**1tp$_{inv}$**), SEQ ID NO: 15 **(4tp),** SEQ ID NO: 27 **(4tp$_{inv}$)**, SEQ ID NO: 16 **(6tp),** and SEQ ID NO: 28 **(6tp$_{inv}$),** and
   - the hexapeptide derivative consists of an amino acid sequence selected from said group, wherein the amino acid sequence comprises a single conservative amino acid substitution, or wherein the amino acid sequence comprises conservative amino acid substitutions at two of positions 1, 3 and 5 or at two of positions 2, 4 and 6.

2. An inhibitory peptide for the use according to claim 1, wherein the inhibitory peptide consists of, or comprises, a hexapeptide or a derivative thereof, wherein:

   - the hexapeptide consists of an amino acid sequence selected from the group consisting of SEQ ID NO: 6 **(4p),** SEQ ID NO: 18 **(4p$_{inv}$)**,**SEQ** ID NO: 7 **(5p)**, SEQ ID NO: 19 (**5p$_{inv}$**), SEQ ID NO: 14 (**1tp**), SEQ ID NO: 26 (**1tp$_{inv}$**), SEQ ID NO: 15 **(4tp)**, SEQ ID NO: 27 **(4tp$_{inv}$)**, SEQ ID NO: 16 **(6tp),** and SEQ ID NO: 28 **(6tp$_{inv}$),** and
   - the hexapeptide derivative consists of an amino acid sequence selected from said group, wherein the amino acid sequence comprises a single conservative amino acid substitution, or wherein the amino acid sequence comprises conservative amino acid substitutions at two of positions 1, 3 and 5 or at two of positions 2, 4 and 6.

3. An inhibitory peptide for the use according to claim 1, wherein the inhibitory peptide consists of, or comprises, a hexapeptide or a derivative thereof, wherein:

   - the hexapeptide consists of an amino acid sequence selected from the group consisting of SEQ ID NO: 6 **(4p),** SEQ ID NO: 7 **(5p)**, SEQ ID NO: 14 (**1tp**), SEQ ID NO: 15 **(4tp),** and SEQ ID NO: 16 **(6tp),** and
   - the hexapeptide derivative consists of an amino acid sequence selected from said group, wherein the amino acid sequence comprises a single conservative amino acid substitution, or wherein the amino acid sequence comprises conservative amino acid substitutions at two of positions 1, 3 and 5 or at two of positions 2, 4 and 6.

4. An inhibitory peptide for the use according to claim 1, wherein the inhibitory peptide consists of, or comprises, a hexapeptide or a derivative thereof, wherein:

- the hexapeptide consists of an amino acid sequence selected from the group consisting of SEQ ID NO: 14 (**1tp**), and SEQ ID NO: 16 **(6tp),** and
- the hexapeptide derivative consists of an amino acid sequence selected from said group, wherein the amino acid sequence comprises a single conservative amino acid substitution, or wherein the amino acid sequence comprises conservative amino acid substitutions at two of positions 1, 3 and 5 or at two of positions 2, 4 and 6.

5. An inhibitory peptide for the use according to any of claims 1 to 4, wherein the hexapeptide or hexapeptide derivative is linked to a heterologous moiety selected from the group consisting of detectable moieties, cell penetrating agents, stability enhancing moieties, tags allowing cellular degradation, and blood-brain barrier shuttle moieties.

6. An inhibitory peptide for the use according to claim 5, wherein the hexapeptide or hexapeptide derivative is linked to a detectable moiety and to a heterologous moiety selected from the group consisting of cell penetrating agents, tags allowing cellular degradation, stability enhancing moieties, and blood-brain barrier shuttle moieties

7. An inhibitory peptide for the use according to claim 5 or claim 6, wherein the hexapeptide or hexapeptide derivative is linked to a detectable moiety suitable for detection by positron emission tomography (PET), in particular to a radionuclide selected from the group consisting of $^{11}$C, $^{13}$N, $^{15}$O, $^{18}$F, $^{62}$Cu, $^{64}$Cu, $^{67}$Cu, $^{67}$Ga , $^{68}$Ga , $^{44}$Sc , $^{43}$Sc, $^{47}$Sc, $^{124}$I, $^{76}$Br, and $^{82}$Rb.

8. An inhibitory peptide for the use according to claim 5 or claim 6, wherein the hexapeptide or hexapeptide derivative is linked to a detectable moiety, and the detectable moiety is a multimodal nanoparticle.

9. An inhibitory peptide for the use according to any of claims 1 to 8, wherein the hexapeptide or hexapeptide derivative is cyclized.

10. A combination of at least two inhibitory peptides as defined in any one of claims 1 to 9 for use in the diagnosis and/or treatment of a tauopathy or a disorder associated with abnormal Tau aggregation in a subject.

11. A pharmaceutical composition comprising at least one inhibitory peptide as defined in any one of claims 1 to 9 and at least one pharmaceutically acceptable carrier or excipient for use in the diagnosis and/or treatment of a tauopathy or a disorder associated with abnormal Tau aggregation in a subject.

12. A pharmaceutical composition for the use according to claim 11, further comprising at least one additional biologically active agent.

13. An inhibitory peptide as defined in any one of claims 1 to 9, or a combination as defined in claim 10 or a pharmaceutical composition as defined in claim 11 or 12, wherein the diagnosis is diagnosis of an early stage of a tauopathy.

14. An inhibitory peptide as defined in any one of claims 1 to 9, or a combination as defined in claim 10 or a pharmaceutical composition as defined in claim 11 or 12, wherein the diagnosis is used to monitor the effectiveness of a tauopathy treatment in a patient.

15. An inhibitory peptide for the use according to any one of claims 1-9 and 13-14, or a combination for the use according to any one of claim 10 and 13-14 or a pharmaceutical composition for the use according to claim 11-14, wherein the tauopathy is selected from the group consisting of Alzheimer's disease (AD), Pick's disease (PD), progressive supranuclear palsy (PSP), corticobasal degeneration (CBD), argyrophilic grain disease (AGD), globular glial tauopathy (GGT), aging-related Tau astrogliopathy (ARTAG), frontotemporal dementia and parkinsonism linked to chromosome 17 (FTDP-17), subacute sclerosing panencephalitis (SSPE), Guadeloupean parkinsonism, Western pacific amyotrophic lateral sclerosis and parkinsonism-dementia complex (ALS/PDC), chronic traumatic encephalopathy (CTE), Huntington Disease (HD); Niemann-Pick disease type C (NPC); Down syndrome; post-encephalitic parkinsonism (PEP), and myotonic dystrophies (types 1 and 2), in particular Alzheimer's disease (AD) and Pick's disease (PD).

Figure 1(A)

**B**

**Tau folding in PHF
Alzheimer's Disease**

**C**

**Tau folding in NPF
Pick's Disease**

**Figure 1(B-C)**

Figure 2

**Figure 3**

**Figure 4**

**Figure 5 (A-B)**

**Figure 5(C)**

**Figure 6**

**Figure 7**

**A**

**B**

**Figure 8**

A

B

**Figure 9**

Europäisches
Patentamt

European
Patent Office

Office européen
des brevets

# EUROPEAN SEARCH REPORT

**Application Number**

EP 21 21 1241

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/039074 A2 (UNIV LELAND STANFORD JUNIOR [US]) 13 March 2014 (2014-03-13) * see claims and examples * | 1-15 | INV. C07K14/47 C07K7/06 A61K38/08 A61K38/17 A61P25/28 G01N33/68 |
| X | WO 2020/232440 A1 (UNIV CALIFORNIA [US]) 19 November 2020 (2020-11-19) * see claims and pages 88-89 * | 1-15 | |
| X | ZHANG XIANCHENG ET AL: "-Enantiomeric Peptide Specifically Binding to PHF6 for Inhibiting Tau Aggregation in Transgenic Mice", ACS CHEMICAL NEUROSCIENCE, vol. 11, no. 24, 7 December 2020 (2020-12-07), pages 4240-4253, XP055935512, US ISSN: 1948-7193, DOI: 10.1021/acschemneuro.0c00518 * see abstract and table 1 * | 1-15 | |
| X | WO 2018/170324 A1 (UNIV CALIFORNIA [US]) 20 September 2018 (2018-09-20) * see claims * | 1-15 | TECHNICAL FIELDS SEARCHED (IPC) C07K A61K G01N A61P |
| T | AILLAUD ISABELLE ET AL: "Tau Aggregation Inhibiting Peptides as Potential Therapeutics for Alzheimer Disease", CELLULAR AND MOLECULAR NEUROBIOLOGY , 21 May 2022 (2022-05-21), XP093027611, US ISSN: 0272-4340, DOI: 10.1007/s10571-022-01230-7 Retrieved from the Internet: URL:https://link.springer.com/article/10.1 007/s10571-022-01230-7/fulltext.html * the whole document * | | |

The present search report has been drawn up for all claims

| Place of search | Date of completion of the search | Examiner |
|---|---|---|
| Munich | 24 March 2023 | Merckling-Ruiz, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 21 1241

This annex lists the patent family members relating to the patent documents cited in the above-mentioned European search report.
The members are as contained in the European Patent Office EDP file on
The European Patent Office is in no way liable for these particulars which are merely given for the purpose of information.

24-03-2023

| Patent document cited in search report | | Publication date | Patent family member(s) | | Publication date |
|---|---|---|---|---|---|
| WO 2014039074 | A2 | 13-03-2014 | CA | 2883447 A1 | 13-03-2014 |
| | | | EP | 2895186 A2 | 22-07-2015 |
| | | | US | 2014161786 A1 | 12-06-2014 |
| | | | WO | 2014039074 A2 | 13-03-2014 |
| WO 2020232440 | A1 | 19-11-2020 | NONE | | |
| WO 2018170324 | A1 | 20-09-2018 | EP | 3596102 A1 | 22-01-2020 |
| | | | US | 2020017563 A1 | 16-01-2020 |
| | | | WO | 2018170324 A1 | 20-09-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018005867 A

**Non-patent literature cited in the description**

- *Alzheimer & Dementia, 2021 Alzheimer's Disease Facts and Figures,* 2021, vol. 17 (3), 327-496
- HUTTON et al. *Nature,* 1998, vol. 393, 702-705
- ISEKI et al. *Acta Neuropathol.,* 2003, vol. 105, 265-270
- QUERFURTH et al. *N. Engl. J. Med.,* 2010, vol. 362, 329-344
- IRWIN et al. *Parkinsonism Relat. Disord.,* 2016, vol. 22, S29-S33
- SILVA et al. *eLife,* 2019, vol. 8, e45457
- GRAHAM et al. *Annu. Rev. Med.,* vol. 2107 (68), 413-430
- MEDINA et al. *Int. J. Mol. Sci.,* 2018, vol. 19, 1160
- CONGDON et al. *Nature Rev. Neurol.,* 2018, vol. 14, 399-415
- JADHAV et al. *Acta Neuropathol. Commun.,* 2019, vol. 7, 22
- VANDEVREDE et al. *Neurosci. Lett.,* 2020, vol. 731, 124919
- NELSON et al. *J. Neuropathol. Exp. Neurol.,* 2012, vol. 71, 362-381
- BARGHORN et al. *Biochem.,* 2004, vol. 43, 1694-1703
- VON BERGEN et al. *J. Biol. Chem.,* 2001, vol. 276, 48165-48174
- GONG et al. *Drugs Aging,* 2010, vol. 27 (5), 351-365
- BRUNDEN et al. *Exp. Neurol.,* 2010, vol. 223 (2), 304-310
- BOUTAJANGOUT et al. *Gerontology,* 2014, vol. 60 (5), 381-385
- BAKOTA et al. *Drugs,* 2016, vol. 76 (3), 301-313
- CONGDON et al. *Nat. Rev. Neurol.,* 2018, vol. 14 (7), 399-415
- CHONG et al. *Cell. Mol. Neurobiol.,* 2018, vol. 38 (5), 965-980
- HOSKIN et al. *Expert Opin. Investig. Drugs,* 2019, vol. 28 (6), 545-554
- DOMINGUEZ-MEIJIDE et al. *Brain Sci.,* 2020, vol. 10 (11), 858
- JOUANNE et al. *Eur. J. Med. Chem.,* 2017, vol. 139, 153-167
- CISEK et al. *Curr. Alzheimer's Res.,* 2014, vol. 11, 918-927
- SEIDLER et al. *Nature Chem.,* 2018, vol. 10, 170-176
- SEIDLER et al. *Nature Chem.,* 2018, vol. 10
- UVERSKY et al. *Biochim. Biophys. Acta (BBA) Proteins Proteom.,* 2013, vol. 1834, 932-951
- MARTINELLI et al. *Int. J. Mol. Sci.,* 2019, vol. 20, 1322
- DOWDY ; WEARDEN. Statistics for Research. John Wiley & Sons, 1983
- E.W. MARTIN. Remington's Pharmaceutical Sciences. Mack Publishing Co, 1990
- KALRA et al. 10. Nanoparticles in Medical Imaging. *Nanoparticles in Analytical and Medical Devices,* 2021, 175-210
- JARZYNA et al. *Rev. Nanomed. Nanobiotechnol.,* 2010, vol. 2 (2), 138-150
- HUANG et al. *Dalton Trans.,* 2011, vol. 40 (23), 6087-6103
- MADRU et al. *J. Nucl. Med.,* 2012, vol. 53 (3), 459
- TANG et al. *Nanomedicine,* 2015, vol. 10 (8), 1343-1359
- BURKE et al. *Philos. Trans. A Math. Phys. Eng. Sci.,* 2017, vol. 375 (2107), 20170261
- Design and Applications of Nanoparticles in Biomedical Imaging. 2017, 205-228
- SCHWARTZ et al. *Curr. Opin. Mol. Ther.,* 2000, vol. 2 (2), 162-7
- LEE et al. *Methods Mol. Biol.,* 2013, vol. 991, 281-92
- BECHARA et al. *FEBS Lett.,* 2013, vol. 587 (12), 1693-1702
- DI PISA et al. *J. Pept. Sci.,* 2015, vol. 21 (5), 356-369
- GUO et al. *Biomed. Rep.,* 2016, vol. 4 (5), 528-534
- SVENSEN et al. *Trends in Pharmacological Sciences,* 2012, vol. 33 (4), 186-192
- VERONESE. *Biomaterials,* 2001, vol. 22, 405-417

- **DEMEULE et al.** *J. Pharmacol. Exp. Ther.,* 2008, vol. 324, 1064-1072
- **DEMEULE et al.** *J. Neurochem.,* 2008, vol. 106, 1534-1544
- **YING et al.** *Angew. Chem., Int. Ed.,* 2014, vol. 53, 12436-12440
- **SPENCER et al.** *Proc. Natl. Acad. Sci. U.S.A.,* 2007, vol. 104, 7594-7599
- **SORRENTINO et al.** *EMBO Mol. Med.,* 2013, vol. 5, 675-690
- **WANG et al.** *Proc. Natl. Acad. Sci. U. S. A.,* 2013, vol. 110, 2999-3004
- **BÖCKENHOFF et al.** *J. Neurosci.,* 2014, vol. 34, 3122-3129
- **RE et al.** *Nanomedicine,* 2011, vol. 7, 551-559
- **MALCOR et al.** *J. Med. Chem.,* 2012, vol. 55, 2227-2241
- **LEE et al.** *Eur. J. Biochem.,* 2001, vol. 268, 2004-2012
- **PRADES et al.** *Biomaterials,* 2012, vol. 33, 7194-7205
- **PRADES et al.** *Angew. Chem., Int. Ed.,* 2015, vol. 54, 3967-3972
- **STAQUICINI et al.** *J. Clin. Invest.,* 2011, vol. 121, 161-173
- **BARRETT et al.** *Regul. Pept.,* 2009, vol. 155, 55-61
- **LIU et al.** *Biomaterials,* 2010, vol. 31, 5246-5257
- **KUMAR.** *Nature,* 2007, vol. 448, 39-43
- **KIM et al.** *Mol. Ther.,* 2010, vol. 18, 993-1001
- **ZADRAN et al.** *NeuroMol. Med.,* 2013, vol. 15, 74-81
- **WEI et al.** *Angew. Chem., Int. Ed.,* 2015, vol. 54, 3023-3027
- **OLLER-SALVIA et al.** *Biopolymers,* 2013, vol. 100, 675-686
- **WU et al.** *Mol. Pharmaceutics,* 2014, vol. 11, 3210-3222
- **OLLER-SALVIA et al.** *Angew. Chem., Int. Ed.,* 2016, vol. 55, 572-575
- **GAILLARD et al.** *J. Controlled Release,* 2012, vol. 164, 364-369
- **MDZINARISHVILI et al.** *Drug Delivery Transl. Res.,* 2013, vol. 3, 309-317
- **LEE et al.** *J. Neuroimmunol.,* 2014, vol. 274, 96-101
- **LINDQVIST et al.** *Mol. Pharmaceutics,* 2013, vol. 10, 1533-1541
- **GEORGIEVA et al.** *Angew. Chem., Int. Ed.,* 2012, vol. 51, 8339-8342
- **COSTANTINO et al.** *J. Controlled Release,* 2005, vol. 108, 84-96
- **TOSI et al.** *Curr. Med. Chem.,* 2013, vol. 20, 2212-2225
- **TOSI et al.** *J. Neural Transm.,* 2011, vol. 118, 145-153
- **VILELLA et al.** *J. Controlled Release,* 2014, vol. 174, 195-201
- **LI et al.** *Biomaterials,* 2011, vol. 32, 4943-4950
- **GAO et al.** *Biomaterials,* 2012, vol. 33, 5115-5123
- **ZHANG et al.** *Biomaterials,* 2014, vol. 35, 456-465
- **SCHWARZE et al.** *Science,* 1999, vol. 285, 156915-72
- **AARTS et al.** *Science,* 2002, vol. 298, 846-850
- **WANG et al.** *Biomaterials,* 2010, vol. 31, 2874-2881
- **ROUSSELLE et al.** *Mol. Pharmacol.,* 2000, vol. 57, 679-686
- **DRIN et al.** *J. Biol. Chem.,* 2003, vol. 278, 31192-3120
- **TEIXIDÓ et al.** *J. Am. Chem. Soc.,* 2007, vol. 129, 11802-11813
- **TEIXIDÓ et al.** *Biopolymers,* 2013, vol. 100, 662-674
- **ARRANZ-GIBERT et al.** *J. Am. Chem. Soc.,* 2015, vol. 137, 7357-7364
- **OLLER-SALVIA et al.** *Chem. Soc. Rev.,* 2016, vol. 45, 4690-4707
- **DIAZ-PERLAS et al.** *Chem. Sci.,* 2018, vol. 9, 8409-8415
- **MAJEROVA et al.** *Molecules,* 2020, vol. 25 (4), 874
- **R.B. MERRIFIELD.** *J. Am. Chem. Soc.,* 1963, vol. 85, 2149-2154
- Solid Phase Peptide Synthesis. Methods in Enzymology. Academic Press, 1997
- **H. NEURATH et al.** The Proteins. Academic Press, 1976, vol. II, 105-237
- **SAMBROOK et al.** Molecular Cloning: A Laboratory Manual. Cold Spring Harbor Press, 2001
- **AUSUBEL et al.** Current Protocols in Molecular Biology. Greene Publishing Associates and John Wiley & Sons, 1994
- **M.P. EDGE et al.** *Nature,* 1981, vol. 292, 756-762
- **SAMBROOK et al.** Molecular Cloning: A Laboratory Manual. Cold Spring Harbor Press, 1989
- **R. KAUFMAN.** *Methods in Enzymology,* 1990, vol. 185, 537-566
- **SHI et al.** *Sci. Transl. Med.,* 2012, vol. 4 (124), 124ra29
- **IOVINO et al.** *Brain,* 2015, vol. 138 (11
- **WRAY.** *Brain Pathl.,* 2017, vol. 27 (4), 525-529
- **ARBER et al.** *Alzheimer's Research & Therapy,* 2017, vol. 9, 42
- **GARCIA-LEON et al.** *Alzheimers Dement.,* 2018, vol. 14 (10), 1261-1280
- **KARCH et al.** *Stem Cell Reports,* 2019, vol. 13 (5), 939-955

- **LIN et al.** *Pharmaceuticals,* 2021, vol. 14 (6), 525
- **PENNEY et al.** *Molecular Psychiatry,* 2020, vol. 25, 148-167
- **KIM et al.** *Nature Protoc.,* 2015, vol. 10 (7), 985-100
- **KWAK et al.** *Nature Commun.,* 2020, vol. 11, 1377
- **DUJARDIN et al.** *Neuropathol. Appl. Neurobiol.,* 2015, vol. 41 (1), 59-80
- **COMBS et al.** *Methods Mol. Biol.,* 2016, vol. 1382, 339-366
- **CUBINKOVA et al.** *Acta Virol.,* 2017, vol. 61 (1), 13-21
- **GÖTZ et al.** *Adv. Exp. Med. Biol.,* 2019, vol. 1184, 381-391
- **GOODARZI et al.** *Cell Tissue Bank,* 2019, vol. 20 (2), 141-151
- *Alzheimers Dement. (NY),* 2020, vol. 6 (1), e12114
- **GIONG et al.** *Int. J. Mol. Sci.,* 2021, vol. 22 (16), 8465
- **CLAIRE SCHIRMER.** Chaperons moléculaires et tauophathies: effet de Hsp90 sur la fibrillation in vitro du peptide VQIVYK issu de la protéine tau. Université de Rennes, 2014
- **GARNIER et al.** *Sci. Rep.,* 2017, vol. 7, 6812

## SEQUENCE LISTING

<110> Université de Rennes
Cyrille, GARNIER

<120> Inhibitory Peptides for the Diagnostic and/or Treatment of Tauopathies

<130> B21101237EP

<160> 51

<170> PatentIn version 3.5

<210> 1
<211> 6
<212> PRT
<213> Homo sapiens

<400> 1

Val Gln Ile Ile Asn Lys
1               5


<210> 2
<211> 6
<212> PRT
<213> Homo sapiens

<400> 2

Val Gln Ile Val Tyr Lys
1               5


<210> 3
<211> 5
<212> PRT
<213> Homo sapiens

<400> 3

His Lys Leu Thr Phe
1               5


<210> 4
<211> 6
<212> PRT
<213> Homo sapiens

<400> 4

Gln Val Glu Val Lys Ser
1               5


<210> 5
<211> 6
<212> PRT
<213> Artificial Sequence

```
<220>
<223>   Peptide 3p

<400>   5

Val Lys Tyr Gln Val Ile
1                   5


<210>   6
<211>   6
<212>   PRT
<213>   Artificial Sequence

<220>
<223>   Peptide 4p

<400>   6

Val Gln Lys Ile Tyr Val
1                   5


<210>   7
<211>   6
<212>   PRT
<213>   Artificial Sequence

<220>
<223>   Peptide 5p

<400>   7

Ile Lys Gln Val Tyr Val
1                   5


<210>   8
<211>   6
<212>   PRT
<213>   Artificial Sequence

<220>
<223>   Peptide 6p

<400>   8

Val Val Gln Lys Tyr Ile
1                   5


<210>   9
<211>   6
<212>   PRT
<213>   Artificial Sequence

<220>
<223>   Peptide 8p

<400>   9

Gln Lys Tyr Ile Val Val
1                   5
```

```
<210>    10
<211>    6
<212>    PRT
<213>    Artificial Sequence

<220>
<223>    Peptide 9p

<400>    10

Ile Tyr Val Gln Lys Val
1               5


<210>    11
<211>    6
<212>    PRT
<213>    Artificial Sequence

<220>
<223>    Peptide 13p

<400>    11

Tyr Ile Val Val Gln Lys
1               5


<210>    12
<211>    6
<212>    PRT
<213>    Artificial Sequence

<220>
<223>    Peptide 14p

<400>    12

Lys Val Gln Tyr Val Ile
1               5


<210>    13
<211>    6
<212>    PRT
<213>    Artificial Sequence

<220>
<223>    Peptide 23 p

<400>    13

Gln Val Ile Lys Tyr Val
1               5


<210>    14
<211>    6
<212>    PRT
<213>    Artificial Sequence
```

```
<220>
<223>   Peptide 1tp

<400>   14

Val Lys Ile Val Tyr Gln
1                   5


<210>   15
<211>   6
<212>   PRT
<213>   Artificial Sequence

<220>
<223>   Peptide 4tp

<400>   15

Val Lys Ile Gln Tyr Val
1                   5


<210>   16
<211>   6
<212>   PRT
<213>   Artificial Sequence

<220>
<223>   Peptide 6tp

<400>   16

Tyr Gln Ile Val Val Lys
1                   5


<210>   17
<211>   6
<212>   PRT
<213>   Artificial Sequence

<220>
<223>   Peptide 3pinv

<400>   17

Ile Val Gln Tyr Lys Val
1                   5


<210>   18
<211>   6
<212>   PRT
<213>   Artificial Sequence

<220>
<223>   Peptide 4pinv

<400>   18

Val Tyr Ile Lys Gln Val
1                   5
```

```
<210>  19
<211>  6
<212>  PRT
<213>  Artificial Sequence

<220>
<223>  Peptide 5pinv

<400>  19

Val Tyr Val Gln Lys Ile
1                   5
```

```
<210>  20
<211>  6
<212>  PRT
<213>  Artificial Sequence

<220>
<223>  Peptide 6pinv

<400>  20

Ile Tyr Lys Gln Val Val
1                   5
```

```
<210>  21
<211>  6
<212>  PRT
<213>  Artificial Sequence

<220>
<223>  Peptide 8pinv

<400>  21

Val Val Ile Tyr Lys Gln
1                   5
```

```
<210>  22
<211>  6
<212>  PRT
<213>  Artificial Sequence

<220>
<223>  Peptide 9pinv

<400>  22

Val Lys Gln Val Tyr Ile
1                   5
```

```
<210>  23
<211>  6
<212>  PRT
<213>  Artificial Sequence
```

<220>
<223>  Peptide 13pinv

<400>  23

Lys Gln Val Val Ile Tyr
1               5

<210>  24
<211>  6
<212>  PRT
<213>  Artificial Sequence

<220>
<223>  Peptide 14pinv

<400>  24

Ile Val Tyr Gln Val Lys
1               5

<210>  25
<211>  6
<212>  PRT
<213>  Artificial Sequence

<220>
<223>  Peptide 23pinv

<400>  25

Val Tyr Lys Ile Val Gln
1               5

<210>  26
<211>  6
<212>  PRT
<213>  Artificial Sequence

<220>
<223>  Peptide 1tpinv

<400>  26

Gln Tyr Val Ile Lys Val
1               5

<210>  27
<211>  6
<212>  PRT
<213>  Artificial Sequence

<220>
<223>  Peptide 4tpinv

<400>  27

Val Tyr Gln Ile Lys Val
1               5

```
<210>  28
<211>  6
<212>  PRT
<213>  Artificial Sequence

<220>
<223>  Peptide 6tpinv

<400>  28

Lys Val Val Ile Gln Tyr
1                   5


<210>  29
<211>  6
<212>  PRT
<213>  Artificial Sequence

<220>
<223>  Peptide 1p

<400>  29

Val Gln Ile Val Tyr Lys
1                   5


<210>  30
<211>  6
<212>  PRT
<213>  Artificial Sequence

<220>
<223>  Peptide 2p

<400>  30

Lys Tyr Ile Gln Val Val
1                   5


<210>  31
<211>  6
<212>  PRT
<213>  Artificial Sequence

<220>
<223>  Peptide 7p

<400>  31

Val Lys Val Ile Tyr Gln
1                   5


<210>  32
<211>  6
<212>  PRT
<213>  Artificial Sequence
```

```
<220>
<223>   Peptide 9p

<400>   32

Ile Tyr Val Gln Lys Val
1                   5


<210>   33
<211>   6
<212>   PRT
<213>   Artificial Sequence

<220>
<223>   Peptide 10p

<400>   33

Lys Val Gln Ile Val Tyr
1                   5


<210>   34
<211>   6
<212>   PRT
<213>   Artificial Sequence

<220>
<223>   Peptide 11p

<400>   34

Val Lys Val Tyr Ile Gln
1                   5


<210>   35
<211>   6
<212>   PRT
<213>   Artificial Sequence

<220>
<223>   Peptide 12p

<400>   35

Val Gln Val Ile Tyr Lys
1                   5


<210>   36
<211>   6
<212>   PRT
<213>   Artificial Sequence

<220>
<223>   Peptide 15p

<400>   36

Lys Tyr Val Ile Gln Val
1                   5
```

```
<210>  37
<211>  6
<212>  PRT
<213>  Artificial Sequence

<220>
<223>  Peptide 16p

<400>  37

Tyr Gln Val Lys Ile Val
1               5


<210>  38
<211>  6
<212>  PRT
<213>  Artificial Sequence

<220>
<223>  Peptide 17p

<400>  38

Tyr Gln Val Ile Val Lys
1               5


<210>  39
<211>  6
<212>  PRT
<213>  Artificial Sequence

<220>
<223>  Peptide 18p

<400>  39

Gln Lys Val Val Tyr Ile
1               5


<210>  40
<211>  6
<212>  PRT
<213>  Artificial Sequence

<220>
<223>  Peptide 19p

<400>  40

Gln Lys Val Tyr Ile Val
1               5


<210>  41
<211>  6
<212>  PRT
<213>  Artificial Sequence
```

```
<220>
<223>   Peptide 20p

<400>   41

Lys Gln Ile Val Tyr Val
1               5


<210>   42
<211>   6
<212>   PRT
<213>   Artificial Sequence

<220>
<223>   Peptide 21p

<400>   42

Val Gln Val Lys Tyr Ile
1               5


<210>   43
<211>   6
<212>   PRT
<213>   Artificial Sequence

<220>
<223>   Peptide 22p

<400>   43

Val Gln Val Tyr Lys Ile
1               5


<210>   44
<211>   6
<212>   PRT
<213>   Artificial Sequence

<220>
<223>   Peptide 0tp

<400>   44

Val Gln Ile Lys Tyr Val
1               5


<210>   45
<211>   6
<212>   PRT
<213>   Artificial Sequence

<220>
<223>   Peptide 2tp

<400>   45

Val Val Ile Gln Tyr Lys
1               5
```

<210> 46
<211> 6
<212> PRT
<213> Artificial Sequence

<220>
<223> Peptide 3tp

<400> 46

Val Val Ile Lys Tyr Gln
1               5


<210> 47
<211> 6
<212> PRT
<213> Artificial Sequence

<220>
<223> Peptide 5tp

<400> 47

Val Gln Tyr Val Ile Lys
1               5


<210> 48
<211> 6
<212> PRT
<213> Artificial Sequence

<220>
<223> Peptide 7tp

<400> 48

Ile Gln Val Val Tyr Lys
1               5


<210> 49
<211> 6
<212> PRT
<213> Artificial Sequence

<220>
<223> Peptide 8tp

<400> 49

Ile Gln Tyr Val Val Lys
1               5


<210> 50
<211> 6
<212> PRT
<213> Artificial Sequence

```
<220>
<223>  Peptide 9tp

<400>  50

Tyr Gln Val Val Ile Lys
1               5


<210>  51
<211>  11
<212>  PRT
<213>  Artificial Sequence

<220>
<223>  Spacer peptide

<400>  51

Gly Pro Asn Ser Lys Gln Ser Gln Asp Glu Gly
1               5                   10
```